(19) Europäisches Patentamt / European Patent Office / Office européen des brevets

(11) **EP 3 235 421 B1**

(12) **EUROPEAN PATENT SPECIFICATION**

(45) Date of publication and mention of the grant of the patent:
**19.09.2018 Bulletin 2018/38**

(51) Int Cl.:
***A61B 3/10*** *(2006.01)*

(21) Application number: **17164178.0**

(22) Date of filing: **31.03.2017**

(54) **SCANNING LASER OPHTHALMOSCOPE**

LASER-SCANNING-OPHTHALMOSKOP

OPHTALMOSCOPE À BALAYAGE LASER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.03.2016 JP 2016073731**

(43) Date of publication of application:
**25.10.2017 Bulletin 2017/43**

(73) Proprietor: **Nidek Co., Ltd.**
**Gamagori-shi**
**Aichi (JP)**

(72) Inventor: **MIZUNO, Katsuyasu**
**Gamagori**
**Aichi (JP)**

(74) Representative: **Hoefer & Partner Patentanwälte mbB**
**Pilgersheimer Straße 20**
**81543 München (DE)**

(56) References cited:
**EP-A1- 2 901 919     JP-A- 2016 028 687**

**Description**

[Technical Field]

The present disclosure relates to a scanning laser ophthalmoscope which captures a front image of a fundus of a subject's eye.

[Background Art]

In the related art, a scanning laser ophthalmoscope is known as a device which captures a front image of a fundus of a subject's eye. In this device, an opening (confocal diaphragm) is disposed at a fundus conjugate position ahead of a light receiving element on which light reflected from the fundus is incident, thereby restraining noise light from being incident on the light receiving element. For example, Patent Document 1 discloses a device in which a lens system (ocular refractive system) is applied to an objective optical system.

EP 2 901 919 A discloses an SLO including a scanning part, a first objective optical system forming two pivot points and a wide-angle comprising a second objective optical system comprising a lens disposed to an optical axis of the irradiation optical system.

[Related art Document]

[Patent Document]

[Patent Document 1] JP-A-2016-28687

[Summary of the Invention]

[Problem that the Invention is to Solve]

Here, in the device in which the lens system is applied to the objective optical system, if a fundus image needs to be obtained with a larger imaging field angle, reflected light on a lens surface of the objective optical system cannot be removed by the confocal diaphragm. In this regard, it is confirmed that the reflected light is likely to be incident on the light receiving element.

The present disclosure is made in view of the above-described problem in the related art, and a technical object is to provide a scanning laser ophthalmoscope which can satisfactorily obtain a front image of a fundus.

[Means for solving the problem]

According to a first aspect of the present disclosure, there is provided a scanning laser ophthalmoscope including an irradiation optical system that includes a light source, a scanning unit for scanning a fundus of a subject's eye with laser light emitted from the light source, and an objective lens system that is disposed between the subject's eye and the scanning unit so as to form a pivot point around which the laser light guided from the scanning unit pivots in response to an operation of the scanning unit, and a light receiving optical system that shares the objective lens system with the irradiation optical system, and that has a light receiving element for receiving light from the fundus irradiated with the laser light. The objective lens system includes a first lens system for bending the laser light toward the pivot point and a second lens system disposed between the first lens system and the scanning unit. The second lens system has a tilted lens disposed to be tilted to an optical axis of the irradiation optical system, and a lens surface on the light source side in the tilted lens has a curvature radius larger than that of a lens surface on the light source side in the first lens system.

According to a second aspect of the present disclosure, there is provided a scanning laser ophthalmoscope including an irradiation optical system that includes a light source, a scanning unit for scanning a fundus of a subject's eye with laser light emitted from the light source, and an objective lens system that is disposed between the subject's eye and the scanning unit so as to form a pivot point around which the laser light guided from the scanning unit pivots in response to an operation of the scanning unit, and a light receiving optical system that shares the objective lens system with the irradiation optical system, and that has a light receiving element for receiving light from the fundus irradiated with the laser light. The scanning unit has two optical scanners which are disposed at mutually different positions and which have mutually different scanning directions of the laser light. The objective lens system includes a first lens system for bending the laser light toward the pivot point and a second lens system disposed between the first lens system and the scanning unit. The second lens system has a tilted lens disposed to be tilted to an optical axis of the irradiation optical system, and is disposed to be tilted to the optical axis of the irradiation optical system so as to be oriented to cancel an astigmatic difference at the pivot point which is caused by the two optical scanners.

[Advantage of the Invention]

According to the present disclosure, it is possible to satisfactorily obtain a front image of a fundus.

[Brief Description of the Drawings]

[Fig. 1] Fig. 1 is a view illustrating an external configuration of SLO according to the present embodiment.
[Fig. 2] Fig. 2 is a view illustrating an optical system in a main body of SLO.
[Fig. 3] Fig. 3 is a view illustrating an example of an aperture unit which is applicable instead of a confocal diaphragm illustrated in Fig. 2.
[Fig. 4] Fig. 4 is a view schematically illustrating a luminous flux of laser light in the vicinity of a scanning unit.
[Fig. 5] Fig. 5 is a view illustrating an objective lens system according to a comparative example.
[Fig. 6] Fig. 6 is a view illustrating the optical system in a state where a lens attachment is mounted thereon.
[Fig. 7] Fig. 7 is a view illustrating a detailed configuration of a light blocking member.
[Fig. 8] Fig. 8 is a view for describing noise light blocked by the light blocking member in the optical system illustrated in Fig. 2.
[Fig. 9] Fig. 9 is a view illustrating an example of a control system in SLO.
[Fig. 10] Fig. 10 is a flowchart illustrating a schematic imaging operation in SLO.
[Fig. 11] Fig. 11 is a view illustrating a captured image (reflected image) of a fundus in SLO.
[Fig. 12] Fig. 12 is a view illustrating a background image corresponding to Fig. 11.
[Fig. 13] Fig. 13 is a view illustrating a difference image between the fundus image illustrated in Fig. 11 and the background image illustrated in Fig. 12.
[Fig. 14] Fig. 14 is a view for describing a partial background image, and illustrates a region extracted as the partial background image from the background image illustrated in Fig. 12.
[Fig. 15] Fig. 15 is a view illustrating a difference image between the fundus image illustrated in Fig. 11 and the partial background image illustrated in Fig. 14.

[Best Mode for Carrying Out the Invention]

Hereinafter, a scanning laser ophthalmoscope (SLO) according to a typical embodiment of the present disclosure will be described with reference to the drawings. A scanning laser ophthalmoscope (hereinafter, referred to as "SLO") 1 according to the present disclosure is a device which acquires a front image of a fundus by scanning the fundus with laser light and receiving returning light of the laser light returning from the fundus. As an imaging method of SLO 1, in addition to an imaging method using fundus reflected light, a fluorescence imaging method is known. The scanning laser ophthalmoscope according to the present disclosure may be a device integrated with other ophthalmology devices such as an optical coherence tomography (OCT) device and a perimeter.
Unless otherwise specifically described, in the embodiment described below, SLO 1 obtains a fundus image by two-dimensionally scanning an observation surface scans with the laser light focused on a spot, based on an operation of a scanning unit. However, a configuration is not necessarily limited thereto. For example, a technique according to the present disclosure may be applied to a so-called line scan SLO. In this case, based on the operation of the scanning unit, the observation surface is one-dimensionally scanned with a linear laser luminous flux.

<External Configuration of Device>

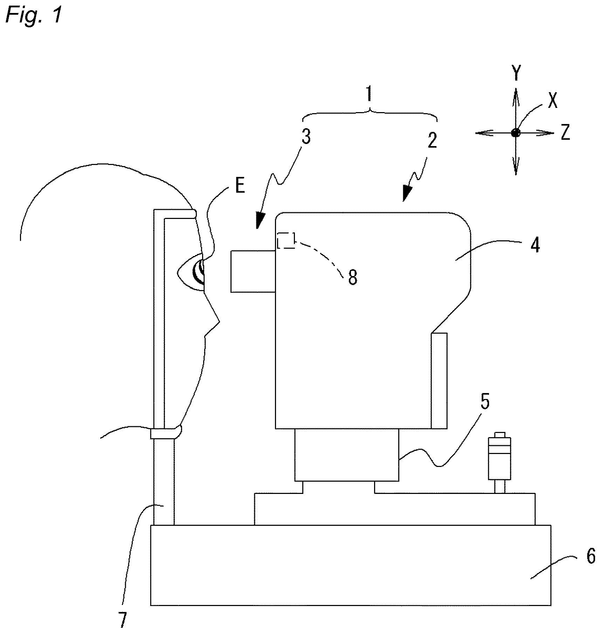

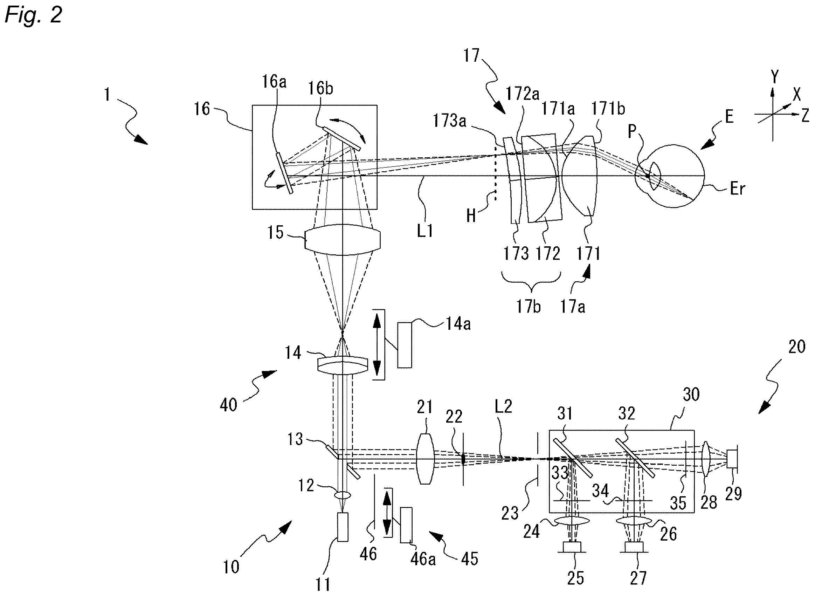

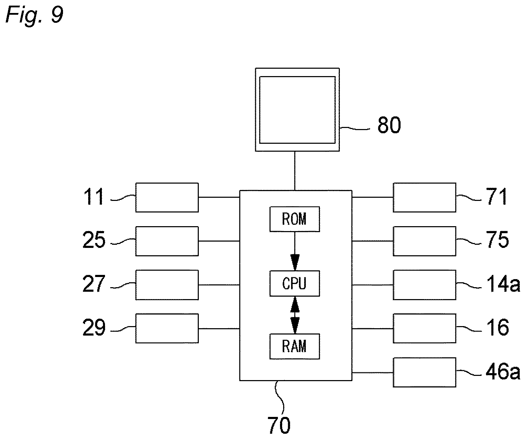

First, referring to Fig. 1, a schematic configuration of the scanning laser ophthalmoscope (SLO) 1 according to the present embodiment will be described. As illustrated in Fig. 1, SLO 1 includes a main body (device body) 2 and a wide angle lens attachment 3 (hereinafter, abbreviated as a "lens attachment 3"). In the present embodiment, the main body 2 has an optical system (refer to Fig. 2) and a control system (refer to Fig. 9) which are mainly used for SLO 1 to image the fundus image. In addition, in the present embodiment, the lens attachment 3 is configured to be attachable to and detachable from a housing 2a of the main body 2. More specifically, the lens attachment 3 is attached to and detached from a surface of the housing at a subject side. SLO 1 can image the fundus in a state where the lens attachment 3 is mounted thereon and in a state where the lens attachment 3 is not mounted thereon, respectively. The lens attachment 3 is mounted on the housing 2a, thereby widening an imaging field angle of the fundus image obtained by the main body 2.
In the present embodiment, the main body 2 has a measurement unit 4, an alignment mechanism 5, a base 6, a face support unit 7, and a sensor 8. The measurement unit 4 stores an optical system for imaging a subject's eye E. This optical system will be described later with reference to Fig 2.

The alignment mechanism 5 is used in order to align the device with the subject's eye E. The alignment mechanism 5 according to the present embodiment three-dimensionally moves the measurement unit 4 to and from the base 6. That is, the measurement unit 4 is moved in each direction of a Y-direction (vertical direction), an X-direction (lateral direction), and a Z-direction (longitudinal direction).

As illustrated in Fig. 1, the face support unit 7 supports a face of the subject in a state where the subject's eye E is caused to face the measurement unit 4. In the present embodiment, the face support unit 7 is disposed in the base 6.

The sensor 8 is detection means for detecting whether the lens attachment 3 is mounted. The sensor 8 outputs an electrical signal corresponding to the mounting state of the lens attachment 3. For example, the sensor 8, may continuously output electrical signals having mutually different voltage values, in a case where the lens attachment 3 is mounted on the main body 2 and in a case where the lens attachment 3 is not mounted on the main body 2. As this sensor 3, a contact sensor such as a micro switch may be used, or for example, a noncontact sensor such as a photoelectric sensor and a magnetic sensor may be used.

<Optical Configuration on Main Body Side>

Next, the optical system disposed in the main body 2 of SLO 1 will be described. As illustrated in Fig. 2, SLO 1 has an irradiation optical system 10 and a light receiving optical system 20 (collectively, referred to as a "photographic optical system"). That is, the irradiation optical system 10 and the light receiving optical system 20 are housed in the same housing 2a. SLO 1 uses these optical systems 10 and 20 so as to capture the fundus image.

The irradiation optical system 10 includes at least a scanning unit 16 and an objective lens system 17. As illustrated in Fig. 2, the irradiation optical system 10 further includes a laser light emitter 11, a collimating lens 12, a perforated mirror 13, a lens 14 (in the present embodiment, a portion of a diopter adjuster 40), and a lens 15.

The laser light emitter 11 is a light source of the irradiation optical system 10. For example, the laser light emitter 11 may include a laser diode (LD) and a super luminescent diode (SLD). Although description of a detailed structure will be omitted, the laser light emitter 11 emits light having at least one wavelength range. In the present embodiment, the light having a plurality of colors is simultaneously or selectively emitted from the laser light emitter 11. For example, in the present embodiment, the light having four colors in total such as three colors of blue, green and red visible ranges and one color in an infrared region is emitted from the laser light emitter 11. For example, the three colors of blue, green and red in the visible range are used for color imaging. For example, the color imaging is performed by substantially simultaneously emitting the light having the three colors of blue, green, and red from the light source 11. Any one color out of the three colors in the visible range may be used for visible fluorescence imaging. For example, blue light may be used for fluorescein angiography (FAG) imaging which is one type of the visible fluorescence imaging. For example, light in the infrared region may be used for infrared fluoroscopy in addition to infrared imaging using the fundus reflected light in the infrared region. For example, as the infrared fluoroscopy, indocyanine green angiography (ICG) imaging is known. In this case, it is preferable that the infrared light emitted from the laser light source 11 is set to have a wavelength range different from the fluorescent wavelength of the indocyanine green angiography imaging.

The laser light is guided to a fundus Er by a light ray path illustrated in Fig. 2. That is, the laser light from the laser light emitter 11 passes through an opening formed in the perforated mirror 13 through the collimating lens 12. After passing through the lens 14 and the lens 15, the laser light travels toward the scanning unit 16. The laser light reflected by the scanning unit 16 passes through the objective lens system 17 and thereafter is used in irradiating the fundus Er of the subject's eye E. As a result, the laser light is reflected and scattered on the fundus Er. Alternatively, the laser light excites a fluorescent material present in the fundus, and causes fluorescence to be detected from the fundus. These types of light (that is, reflected and scattered light and fluorescence) are emitted from a pupil as the returning light.

In the present embodiment, the lens 14 illustrated in Fig. 2 is a portion of the diopter adjuster 40. The diopter adjuster 40 is used in order to correct (reduce) a diopter error of the subject's eye E. For example, the lens 14 is movable in a direction of the optical axis of the irradiation optical system 10 by a drive mechanism 14a. Depending on a position of the lens 14, a diopter of the irradiation optical system 10 and the light receiving optical system 20 is changed. Therefore, the position of the lens 14 is adjusted, thereby reducing the diopter error of the subject's eye E. As a result, a light focusing position of the laser light can be set to an observation site (for example, a retinal surface) of the fundus Er. For example, the diopter adjuster 40 may be applied to an optical system different from that illustrated in FIG. 2, such as a Bardahl optical system.

The scanning unit 16 (also referred to as an "optical scanner") is used in order to scan the fundus with the laser light emitted from the light source (laser light emitter 11). In the following description, unless otherwise specifically described, the scanning unit 16 includes two optical scanners which have mutually different scanning directions of the laser light. The two optical scanners are disposed at mutually different positions. That is, the scanning unit 16 includes an optical scanner 16a for main scanning (for example, for scanning in the X-direction) and an optical scanner 16b for sub-scanning (for scanning in the Y-direction). The optical scanner 16a for main scanning and the optical scanner 16b for sub-scanning may be respectively a resonant scanner and a galvano mirror. However, the invention is not necessarily limited thereto, and in addition to other reflection mirrors (a galvano mirror, a polygon mirror, a resonant scanner or MEMS), an acousto optical modulator (AOM) which changes a light traveling (deflection) direction may be applied to the respective optical scanners 16a and 16b. The scanning unit 16 does not necessarily include a plurality of the optical scanners, and may include one optical scanner. As a single optical scanner which two-dimensionally scans the fundus with the laser light, it is proposed to utilize any one of a MEMS device and an acousto optical modulator (AOM), for example.

The objective lens system 17 serves as an objective optical system of SLO 1. The objective lens system 17 is utilized in order to guide the laser light used in scanning by the scanning unit 16 to the fundus Er. Therefore, the objective lens system 17 forms a pivot point P around which the laser light passing through the scanning unit 16 pivots. The pivot point P is formed on an optical axis L1 of the irradiation optical system 10 at a position which is optically conjugate with the scanning unit 16 with regard to the objective lens system 17. A detailed configuration of the objective lens system 17 will be described later. In the present disclosure, "conjugation" is not necessarily limited to a complete conjugate relationship, but includes "substantial conjugation". That is, even in a case where both of these are disposed by being deviated from a complete conjugate position within an allowable range in relation to a use purpose (for example, observation or analysis) of the fundus image, the meaning is included in the "conjugation" in the present disclosure.

The laser light passes through the objective lens system 17 after passing through the scanning unit 16, thereby causing the fundus Er to be irradiated with the laser light via the pivot point P. Therefore, the laser light passing through the objective lens system 17 is caused to pivot around the pivot point P in response to the operation of the scanning unit 16. As a result, in the present embodiment, the fundus Er is two-dimensionally scanned with the laser light. The laser light used in irradiating the fundus Er is reflected at a light focusing position (for example, a retinal surface). In addition, the laser light is scattered by tissues present in the front and rear of the light focusing position. The reflected light and the scattered light are respectively emitted from a pupil as parallel light.

Next, the light receiving optical system 20 will be described. The light receiving optical system 20 shares the objective lens system 17 with the irradiation optical system 10, and has at least one light receiving element. For example, as illustrated in Fig. 2, the light receiving optical system 20 may have a plurality of light receiving elements 25, 27, and 29. In this case, the light from the fundus Er irradiated with the laser light by the irradiation optical system 10 is received by the light receiving elements 25, 27, and 29.

As illustrated in Fig. 2, the light receiving optical system 20 according to the present embodiment may share each member disposed from the objective lens system 17 to the perforated mirror 13 with the irradiation optical system 10. In this case, the light from the fundus is guided to the perforated mirror (in the present embodiment, an optical path diverging member) 13 through an optical path of the irradiation optical system 10. In addition, the perforated mirror 13 diverges the irradiation optical system 10 and the light receiving optical system 20 from each other. In the present embodiment, the light from the fundus Er is reflected by a reflective surface of the perforated mirror 13 so as to be guided to an independent optical path (optical path on the light receiving elements 25, 27, and 29 sides) of the light receiving optical system 20. The light traveling toward the perforated mirror from an optical path common to the irradiation optical system 10 and the light receiving optical system 20 is likely to include noise light in a region in the vicinity of an optical axis L2 in the principal ray of the laser light. The noise light described herein mainly indicates the light from those other than imaging and observation targets, such as the light focusing position (for example, the retinal surface) of the fundus Er. Specifically, the noise light includes the reflected light from a cornea and the reflected light from the optical system inside the device. The perforated mirror 13 guides the light from the fundus Er to the independent optical path of the light receiving optical system 20 while removing at least a portion of the noise light. In the present embodiment, the perforated mirror 13 is disposed at a position conjugate with an anterior ocular segment. Therefore, the reflected light from the cornea and the reflected light from the optical system inside the device are removed by an opening of the perforated mirror 13. On the other hand, within the light from the fundus Er, the light passing through the periphery of the pupil is reflected on the reflective surface of the perforated mirror 13, and is guided to the independent optical path.

The optical path diverging member which diverges the irradiation optical system 10 and the light receiving optical system 20 from each other is not limited to the perforated mirror 13. For example, instead of the perforated mirror 13, a member replacing the opening and reflective surface with each other in the perforated mirror 13 can be utilized as an optical path diverging unit. However, for example, in a case where the member is applied to Fig. 2, it is further necessary to dispose the independent optical path (from the light source 11 to the perforated mirror 13) of the irradiation optical system 10 and the independent optical path (from the perforated mirror 13 to the light receiving elements 25, 27, and 29) of the light receiving optical system 20 by replacing both of these with each other. As the optical path diverging member, other members such as a beam splitter may be used.

The light receiving optical system 20 has a lens 21, a light blocking unit 22, a pinhole plate 23, and a light separator (light separating unit) 30 in a reflected light path of the perforated mirror 13. In addition, lenses 24, 26, and 28 are disposed between the light separator 30 and each of the light receiving elements 25, 27, and 29.

Although described in detail later, the light blocking unit 22 blocks the light in the vicinity of the optical axis L2 of the light receiving optical system 20. The light from a fundus conjugate plane passes through the light blocking unit 22, and at least a portion of the noise light is blocked by the light blocking unit 22.

In addition, the pinhole plate 23 is disposed on the fundus conjugate plane, and functions as a confocal diaphragm in SLO 1. That is, in a case where the diopter is properly corrected by the diopter adjuster 40, the light from the fundus Er after passing through the lens 21 is focused on the opening of the pinhole plate 23. The light from positions other than a focal point (or a focal plane) of the fundus Er is removed by the pinhole plate 23, and the remaining light (light from the focal point) is mainly guided to the light receiving elements 25, 27, and 29.

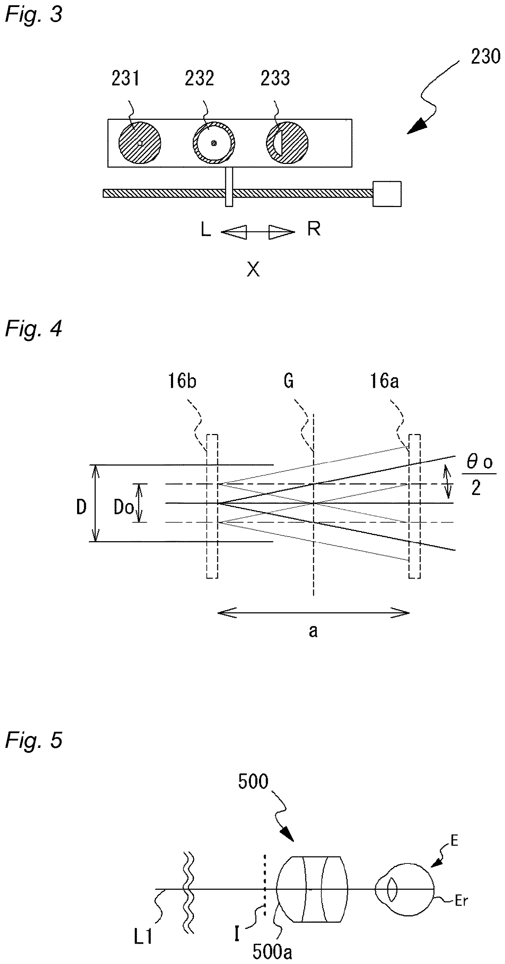

Unless otherwise specifically described, description will be made on the assumption that the pinhole plate 23 (confocal diaphragm) is located at the fundus conjugate position between the light receiving elements 25, 27, and 29 and the perforated mirror 13. However, an aperture unit 230 as illustrated in Fig. 3 may be provided instead of the pinhole plate 23. As the aperture unit 230, any one is provided by switching between a confocal diaphragm 231 having an opening at a position conjugate with the focal point of the observation surface (fundus), and apertures 232 and 232 having a light blocking unit at a position conjugate with the focal point of the observation surface (fundus) and having an opening for allowing the light present in a region away from the optical axis L2 within the light scattered in the front and rear of the focusing point to pass through the light receiving elements 25, 27, and 29, and 232. In a case where the apertures 232 and 232 are provided, the fundus image based on the scattered light from the front and rear of the focal point is acquired. In this case, it is possible to image a minute biological substance in the fundus which cannot be confirmed using the fundus image (for example, a reflected image) captured when the confocal diaphragm 231 is provided.

The light separator 30 separates the light from the fundus Er. In the present embodiment, the light from the fundus Er is separated by the light separator 30 in a wavelength-selective manner. In addition, the light separator 30 may also serve as an optical diverging unit which diverges the optical path of the light receiving optical system 20. For example, as illustrated in Fig. 2, the light separator 30 may include two dichroic mirrors (dichroic filters) 31 and 32 having different light separation characteristics (wavelength separation characteristics). The optical path of the light receiving optical system 20 is diverged into three by the two dichroic mirrors 31 and 32. In addition, each one of the light receiving elements 25, 27, and 29 is disposed in front of each diverged optical path.

For example, the light separator 30 separates the wavelength of the light from the fundus Er, and causes the three light receiving elements 25, 27, and 29 to receive the light having mutually different wavelength ranges. For example, the light having three colors of blue, green, and red may be received one by one by the light receiving elements 25, 27, and 29. In this case, a color image can be easily obtained based on the result of the light received by the light receiving elements 25, 27, and 29.

In addition, the light separator 30 may cause at least one of the light receiving elements 25, 27, and 29 to receive the light in the infrared region used in infrared imaging. In this case, for example, fluorescence used in fluorescence imaging and the light in the infrared region used in the infrared imaging may be received by mutually different light receiving elements.

The wavelength bands where the light receiving elements 25, 27, and 29 are sensitive may be different from each other. In addition, at least two of the light receiving elements 25, 27, and 29 may be sensitive to a common wavelength range. Each of the light receiving elements 25, 27, and 29 outputs a signal corresponding to the intensity of the received light (hereinafter, referred to as a received light signal). In the present embodiment, the received light signal is separately processed by each light receiving element so as to generate an image. That is, in the present embodiment, maximum three types of the fundus image are concurrently generated.

In SLO 1 (here, the optical system of the main body 2), the position conjugate with the anterior ocular segment from the perforated mirror 13 to the objective lens system 17 is formed at only two positions such as the position of the scanning unit 16 and the position of the perforated mirror 13. This realizes the optical system which has a short entire length.

<Condition of Arrangement Interval between Optical Scanners>

If the laser light used in irradiating the fundus is vignetted by the pupil, the fundus image is obtained which is dark around. In contrast, vignetting can be restrained by setting an interval a between the optical scanner 16a and the optical scanner 16b so as to satisfy Expression (1) below.

$$a \leq \{\sin(\theta/2)/\sin(\theta o/2)\} \cdot (k-1) \cdot \phi/\tan(\theta o/2) \qquad (1)$$

Here $\theta$ represents an angle indicating a visual field diameter, $\theta o$ represents a full angle of an optical swing angle in the scanning unit 16 (optical scanners 16a and 16b), and $\phi$ represents an image diameter of the opening of the perforated mirror 13 in the anterior ocular segment. In addition, k represents a constant obtained based on examination made by the inventor, and k is a value greater than 1. Based on the examination made by the inventor, it is confirmed that if the constant k is set to a value of approximately "1.5", the vignetting is less likely to occur even if other parameters are changed.

For example, in a case of $\theta=60°$, $\theta o=16°$, $\phi=1.4$ mm, and k=1.5, if a is within 18 mm, the laser light pivots while passing through the vicinity of the pivot point P. For example, even if the eye has a small pupil diameter of approximately $\phi 2.5$ mm, the laser light is less likely to be vignetted.

Expression (1) will be described with reference to Fig. 4. Fig. 4 is a view schematically illustrating the periphery of the scanning unit 16. Fig. 4 schematically illustrates a luminous flux when the light is emitted to pass through the perforated mirror 13. G represents an intermediate point between the optical scanners 16a and 16b, and is formed at the position conjugate with the anterior ocular segment conjugate (conjugated with the pupil). The thickness of the luminous flux (actual thickness of the luminous flux) in a case where the optical scanners 16a and 16b are stationary is illustrated as Do. In addition, the apparent thickness of the laser light at the intermediate point C (intermediate plane) during operation of the optical scanners 16a and 16b is illustrated by D.

Here, "$\sin(\theta/2)/\sin(\theta o/2)$" in Expression (1) is considered as a term representing the magnification in the objective lens system 17 on the assumption of the sine condition. Therefore, "$\sin(\theta/2)/\sin(\theta o/2)\cdot\phi$" included in Expression (1) is synonymous with the thickness Do of the actual luminous flux. Here, Expression (1) is rewritten using Do so as to obtain Expression (2) below.

$$a \leq Do\cdot(k-1)/\tan(\theta o/2)$$

$$a\cdot\tan(\theta o/2) \leq Do\cdot(k-1) \qquad (2)$$

As is clarified from Fig. 4, a relationship of "$D=Do+a\cdot\tan(\theta o/2)$" is satisfied. Therefore, if Expression (2) is replaced and rearranged, it is possible to derive Expression (3).

$$D=Do+\tan(\theta o/2) \leq Do\cdot k \qquad (3)$$

In this way, Expression (1) serves as a condition for determining that the thickness D of the apparent luminous flux at the intermediate point C which is position conjugate with the anterior ocular segment is within k times the thickness Do of the actual luminous flux. Focuses on this relationship, the inventor confirms that if the constant k is set as a value of approximately "1.5", the vignetting is less likely to occur actually.

(Optical Configuration Contributing to Noise Restraint>

As described above, SLO 1 according to the present embodiment can restrain the noise light from being incident on the light receiving elements 25, 27, and 29 by the confocal diaphragm (pinhole plate 23) and the perforated mirror 13.

However, in general, in SLO having the objective lens system, it is difficult to secure the focal length of the objective lens system with sufficient margin if the imaging field angle needs to be secured up to several tens of degrees or more. In this case, in the objective lens system, the lens surface on the light source side having the lens which greatly bends the laser light is disposed close to the fundus conjugate plane (also, referred to as an intermediate image plane) relating to the objective lens system. In this manner, the noise light which is not removed by the confocal diaphragm and the perforated mirror is likely to be generated. In the lens which greatly bends the laser light, the curvature radius on the lens surface on the light source side tends to become smaller. Therefore, even if the lens is tilted, the noise light is less likely to escape outward from the light receiving optical path. The fundus conjugate plane relating to the objective lens system is a primarily magnified image formed using the objective lens system, and mainly indicates an inverted image. However, the fundus conjugate plane is not necessarily limited to the primarily magnified image.

In some cases, diopter correction is performed in order to properly focus the laser light on the fundus. In this case, the fundus conjugate plane formed via the objective lens system moves in accordance with the amount of the diopter correction. For example, in a case where the myopia of the subject's eye is severe, as a result of the diopter correction, the fundus conjugate plane relating to the objective lens system moves close to the subject's eye E side. Therefore, it is considered that the influence of the diopter correction also generates the noise light which is not removed by the confocal diaphragm and the perforated mirror etc.

In contrast, SLO 1 according to the present embodiment may have configurations described below. When the technique according to the present disclosure is carried out, the following configurations are not necessarily all adopted, and may be partially selectively adopted.

<Objective Lens System>

As illustrated in Fig. 2, the objective lens system 17 has a first lens system 17a and a second lens system 17b. Each of the lens systems 17a and 17b includes at least one or more lenses. The first lens system 17a is a main lens system for bending the laser light toward the pivot point in the objective lens system 17. For example, the first lens system 17a illustrated in Fig. 2 bends the laser light toward the pivot point P at a position where a principal ray height of the laser light becomes highest in the objective lens system 17. In addition, the first lens system 17a is disposed closest to the subject's eye side in the objective lens system 17.

In the present embodiment, the first lens system 17a is formed using one lens 171. As illustrated in Fig. 2, an aspheric lens may be used for the lens 171.

Incidentally, as illustrated in Fig. 2, in order to realize the fundus imaging with an imaging field angle of several tens of degrees or more while sufficiently securing an operating distance between the device and the subject's eye E, a lens having an aperture diameter to some extent needs to be disposed in first lens system 17a so as to bend the laser light. Therefore, an aberration (for example, a spherical aberration and a coma aberration) depending on the aperture diameter of the lens is likely to occur in the objective lens system 17 (particularly, the first lens system 17a). In contrast, it is desirable that the lens 171 has the lens surface formed in an aspheric shape so as to reduce the aberration depending on the aperture diameter of the lens. For example, the lens 171 has the aspheric lens surface so as to reduce the spherical aberration of the image formation at the pivot point P. For the example, the lens surface 171a on the light source side in the lens 171 may be an aspheric surface. For example, the lens surface 171a may be formed using a curved surface so that the curvature radius increases as the position is farther away from the optical axis (the lens axis) of the lens 171. On the other hand, the lens surface 171b on the subject's eye side in the lens 171 may be a spherical surface, a flat surface, or an aspheric surface.

It is preferable that the lens 171 (or the first lens system 17a) is disposed so that the optical axis extends along the optical axis L1 of the irradiation optical system 10. For example, the lens 171 has strong refracting power. Accordingly, the distortion in the fundus image can be restrained by disposing the lens 171 so that the optical axis of the lens 171 extends along the optical axis L1. Here, the optical axis of the lens 171 may be completely coaxial with the optical axis L1 of the irradiation optical system 10, or may be slightly tilted to the optical axis L1. For example, the optical axis L1 of the lens 171 may be slightly tilted so that the distortion of the fundus image is allowed. The lens 171 is disposed so that the optical axis of the lens 171 is tilted to the optical axis L 1. Accordingly, a tilt of the image plane derived from "the tilted lens" (to be described later) may be reduced in some cases.

The second lens system 17b is disposed between the first lens system 17a and the scanning unit 16. The second lens system 17b is configured so that the lens surface on the light source side has at least one lens (referred to as a "tilted lens" for the sake of convenience in the following description) which is tilted to the optical axis L1 of the irradiation optical system 10. If the second lens system 17b includes a plurality of lenses, two or more lenses may serve as the tilted lens, or all of the lenses may serve as the tilted lens as illustrated in Fig. 2.

For example, in the present embodiment, each of the cemented lens 172 and the convex meniscus lens 173 serves as the tilted lens in the second lens system 17b. As illustrated in Fig. 2, in each of the tilted lenses 172 and 173, the lens surfaces 172a and 173a on the light source side are formed to have the larger curvature radius than the lens surface 171a on the light source side in the first lens system 17. Any one of the tilted lenses 172 and 173 illustrated in Fig. 2 is a spherical lens. In addition, any one of the tilted lenses 172 and 173 has positive power. However, the present invention is not necessarily limited thereto, and the aspheric lens and/or the lens having negative power may be applied to some or all of the lenses included in the second lens system 17b.

The second lens system 17b may include the lens for correcting the aberration caused by the first lens system 17a. For example, the cemented lens 172 according to the present embodiment corrects the chromatic aberration. The chromatic aberration to be corrected may be any one of axial chromatic aberration and magnification chromatic aberration, or may be both of these. More specifically, the cemented lens 172 may be an achromatizing lens formed by joining at least two lenses having mutually different light dispersion characteristics (Abbe number) to each other. The laser light is reflected on a joining surface 172b of the cemented lens 172, thereby causing a possibility that the noise light may be generated. In contrast, refractive indexes of each lens (concave lens and convex lens in Fig. 2) included in the cemented lens 172 and an adhesive for joining the respective lenses to each other may coincide with each other (including substantial coincidence). This retrains the laser light from being reflected on the joining surface 172b. As a result, the noise light caused by the reflection on the joining surface 172b is less likely to be generated. Here, if a difference in the refractive indexes between each lens included in the cemented lens 172 and the adhesive is 0.01 or smaller, a restraining effect of the noise light is more preferably obtained.

It is preferable that the joining surface 172b in the cemented lens 171 is convex toward the subject's eye side. The reason is that the noise light is restrained from being generated, since the joining surface 172b and a fundus conjugate plane H are separated from each other.

In addition, as the refractive indexes of each lens included in the cemented lens 172 and the adhesive are higher, it becomes easier to increase the curvature radius of the joining surface, and the reflected light on the joining surface 172b is less likely to be reflected using an angle at which the reflected light is incident on the light receiving elements 25, 27, and 29. As a result, the influence of the noise light on the fundus image is further restrained. In addition, the manufacturing cost of the cemented lens 172 can be restrained by increasing the curvature radius of the joining surface 172b. For example, the concave lens and the convex lens and the adhesive for joining the respective lenses to each other may be selected so that the refractive indexes substantially coincide with each other at 1.63.

The number of the lenses forming the cemented lens 172 may be determined in accordance with the number of colors included in the laser light. For example, in the cemented lens 172 in a double configuration, a light focusing position of the light having two colors among three colors included in the laser light can be replaced with a light focusing position of the light having another one color. As described above, SLO 1 according to the present embodiment emits the light having four colors in total, such as three colors of red, green, and blue and one color in the infrared region, from the laser light emitter 11. In a case of the cemented lens 172 having the double configuration as illustrated in Fig. 2, the light focusing position of the blue light having the shortest wavelength and the light focusing position of the light in the infrared region may be designed so as to coincide with the light focusing position of the light having an intermediate wavelength (here, the "intermediate wavelength" does not always have to coincide with (including substantial coincidence) the wavelength of the laser light emitted from the laser light emitter 11). Since the cemented lens 172 is provided, the position of the pivot point in the laser light of each color is less likely to be deviated. As a result, in the fundus imaging performed by emitting the light having a plurality of colors, the fundus can be satisfactorily imaged for the eye having a smaller pupil or at a larger imaging field angle.

Each lens included in the cemented lens 172 may be selected so that the power of the cemented lens 172 is zero. In this case, the cemented lens 172 is less likely to affect the tilt and the height of the light ray. Therefore, the presence or absence of the cemented lens 172 is less likely to affect the design of the lens (for example, the first lens system 17a) having a role to bend the laser light in the objective lens system 17.

The convex meniscus lens 173 has positive power. Therefore, the convex meniscus lens 173 contributes to the role to bend the laser light to the pivot point P. However, the amount of bending is sufficiently smaller than that of the first lens system 17a.

Incidentally, in order to secure approximately the same imaging field angle as that according to the present embodiment, a design solution is conceivable in which a plurality of lenses included in the objective lens system are formed as one cemented lens 500 (refer to a comparative example in Fig. 5). However, in a case where the objective lens system is formed using one cemented lens 500, a lens surface 500a is likely to be disposed close to the fundus conjugate plane (intermediate image I). As a result, the noise light is likely to be generated by the reflection of the laser light on the lens surface 500a. In addition, the curvature radius of the lens surface 500a on the light source side decreases. Accordingly, even if the lens 500 is tilted, the reflected light on the lens surface 500a is less likely to escape from the light receiving optical path.

Compared to this comparative example, the objective lens system 17 according to the present embodiment is divided into the first lens system 17a and the second lens system 17b. The second lens system 17b is disposed between the first lens system 17a serving as the main lens system for bending the laser light toward the pivot point and the scanning unit 16. In this manner, the lens surface 171a of the first lens system having the relatively small curvature radius in the objective lens system 17 is easily disposed away from the fundus conjugate plane (intermediate image H) relating to the objective lens system 17. As a result, the reflected light on the lens surface 171a is less likely to be incident on the light receiving elements 25, 27, and 29 as the noise light. In addition, in the tilted lenses 172 and 173 of the second lens system 17b, the lens surfaces 172a and 173a on which the laser light is reflected are tilted to the optical axis L1. Furthermore, the lens surfaces 172a and 173a are formed to have the larger curvature radius than that of the lens surface 171a of the first lens system 17a. Accordingly, even if the position of any one of the lens surfaces 172a and 173a is located in the vicinity of the fundus conjugate plane (intermediate image H), the reflected light on the lens surfaces 172a and 173a is less likely to be incident on the light receiving elements 25, 27, and 29 as the noise light. As a result, according to the objective lens system 17 of the present embodiment, the noise light incident on the light receiving elements 25, 27, and 29 is less likely to be generated.

Incidentally, as illustrated in Fig. 2, the optical scanner 16a for main scanning and the optical scanner 16b for sub-scanning are separate from each other. This may be a factor causing an astigmatic difference at an image position of the scanning unit 16 in the pupil of the subject's eye E. That is, it may be a factor causing deviation in the direction extending along the optical axis L1 between the position of the pivot point of the laser light in the main scanning direction and the position of the pivot point of the laser light in the sub-scanning direction. If there is the astigmatic difference, there is a high possibility that the laser light may be vignetted in the pupil. Therefore, unevenness is likely to appear so that the brightness in the peripheral portion of the fundus image is darker compared to the central portion.

In contrast, the tilted lenses 172 and 173 included in the second lens system 17b are disposed to be tilted to the optical axis L1 in a direction so as to cancel (reduce) the astigmatic difference caused by the two optical scanners 16a and 16b. In this case, the astigmatic difference caused by the tilt of the tilted lens and the astigmatic difference caused by the two optical scanners 16a and 16b are mutually canceled. This restrains the astigmatic difference remaining at the pivot point P. For example, as illustrated in Fig. 2, in a case where the optical scanner 16b for sub-scanning, the optical scanner 16a for main scanning are disposed in this order from the light source side, the tilted lens is appropriately tilted within a yz plane (inside the drawing paper). In this manner, the astigmatic difference can be reduced. As a result, the fundus image with uniform brightness is easily obtained.

<Wide Angle Lens Attachment>

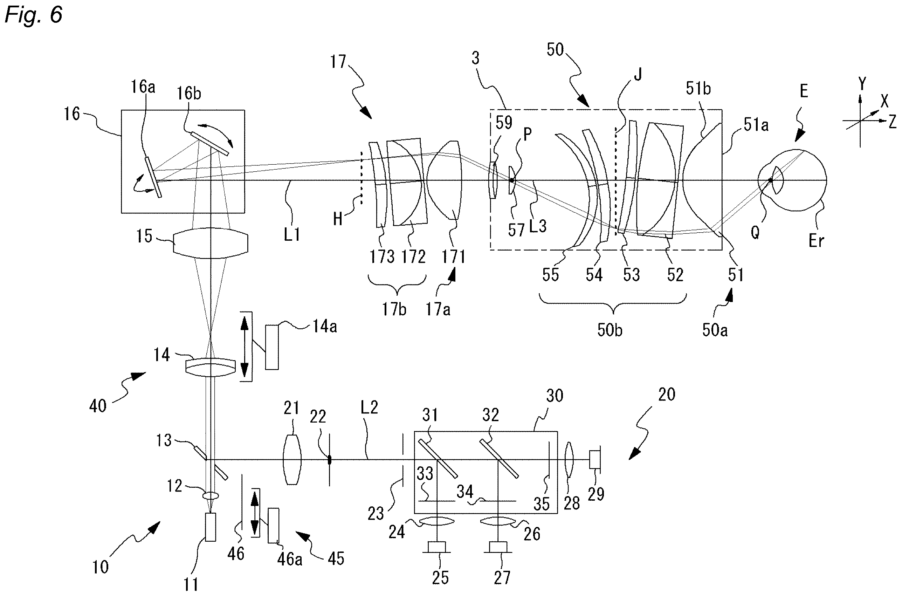

The lens attachment 3 is mounted on SLO 1. Referring to Fig. 6, the optical system in a state where the lens attachment 3 is mounted thereon will be described.

The lens attachment 3 is mounted on the surface of the housing at the subject side of SLO 1 so that the second objective lens system 50 (to be described later) is disposed in the optical path of the laser light. For example, the lens attachment 3 is disposed in the vicinity of the first pivot point P.

As illustrated in Fig. 6, the lens attachment 3 mainly has the second objective lens system 50. In addition to this, the lens attachment 3 may have the lens system and a mirror system. For example, the lens attachment 3 illustrated in Fig. 6 has a diopter correction lens 57 (diopter correction lens optical system). The lens attachment 3 is mounted on SLO 1, thereby allowing the above-described optical system included in the lens attachment 3 to be disposed between the objective lens system 17 of SLO 1 and the subject's eye E.

The diopter correction lens 57 offsets a diopter change caused by the second objective lens system 50 (that is, the diopter change is partially or entirely canceled). More specifically, the diopter correction lens 57 restrains the diopter difference of the light incident on the subject's eye E in a state where the lens attachment 3 is mounted thereon and in a state where the lens attachment 3 is not mounted thereon. As illustrated in Fig. 6, the diopter correction lens 57 may be the lens having positive power (more specifically, a plano-convex lens whose convex surface faces the subject's eye E side). In a case where the fundus imaging needs to be realized at a wide angle (for example, an imaging field angle of $\phi 90°$ or larger) due to the mounted lens attachment 3, it is assumed that the second objective lens system 50 has refractive power of several tens of diopters or more. In this case, a case is conceivable where the body of SLO 1 (for example, the irradiation optical system 10) cannot fully correct the diopter change. In contrast, the lens having strength corresponding to the second objective lens system 50 is employed as the diopter correction lens 57, thereby offsetting the diopter change caused by the second objective lens system 50. As a result, the fundus image can be satisfactorily captured using the wide angle. In addition, according to the diopter correction lens 57, the diopter difference of the light incident on the subject's eye E is reduced in the state where the lens attachment 3 is mounted thereon and in the state where the lens attachment 3 is not mounted thereon. Therefore, when the lens attachment 3 is mounted on or detached from SLO 1, the diopter is conveniently adjusted, or does not need to be adjusted.

In addition, as illustrated in Fig. 6, the diopter correction lens 57 is disposed on the light source side with respect to the second objective lens system 50. More specifically, the diopter correction lens 57 is disposed at the position of the pivot point P. As a result, the laser light passes through the vicinity of the center of the diopter correction lens 57. Accordingly, the lens having a small diameter can be used as the diopter correction lens 57. In addition, since the laser light passes through the vicinity of the center of the diopter correction lens 57, the tilt of the principal ray of the laser light is less likely to be affected by the power of the diopter correction lens 57. As a result, the diopter setting can be changed only by changing the diopter correction lens 57. In this manner, the number of design steps in the second objective lens system 50 is reduced. The diopter correction lens 57 may not necessarily be located at the pivot point P. However, it is desirable to avoid the diopter correction lens 57 from being disposed on the fundus conjugate plane (that is, the intermediate image L, in other words, the primarily magnified image by the second objective lens system 50) inside the lens attachment 3.

The second objective lens system 50 forms a pivot point Q (second pivot point Q) in the state where the lens attachment 3 is mounted thereon. The pivot point Q is a point around which the laser light (more particularly, the primary ray of the laser light) passing through the pivot point P further pivots in response to the operation of the scanning unit 16. The pivot point Q is formed at a position optically conjugate with the scanning unit 16 with regard to the second objective lens system 50. The pivot point Q is formed on an optical axis L3 of the second objective lens system 50. As illustrated in Fig. 6, the optical axis L3 of the second objective lens system 50 may be coaxial with the optical axis L1 of the irradiation optical system 10.

The second objective lens system 50 has a first lens system 50a and a second lens system 50b. The first lens system 50a in the second objective lens system 50 may have at least a portion of the above-described characteristics with regard to the first lens system 17a in the first objective lens system 17. In addition, the second lens system 50b in the second objective lens system 50 may have at least a portion of the above-described characteristics with regard to the second lens system 17b in the first objective lens system 17.

That is, the first lens system 50a is the main lens system for bending the laser light toward the pivot point Q in the second objective lens system 50. That is, the first lens system 50a bends the laser light toward the pivot point Q at the position where the principal ray height of the laser light in the second objective lens system 50 is highest. In addition, the first lens system 50a is disposed closest to the object's eye side in the second objective lens system 50.

The first lens system 50a may be formed using one lens 51. In addition, the aspheric lens for restraining the spherical aberration of the image formation at the pivot point Q may be applied to the lens 51. In order to reduce the spherical aberration, the lens surface 51a on the light source side is formed using a curved surface whose curvature radius increases as the position is farther away from the optical axis L3 of the second objective lens system 50.

Here, the imaging field angle of the fundus image and the operating distance are in a trade-off relationship. For example, in order to secure the larger operating distance while achieving the imaging field angle of φ90° or larger, the lens 51 is formed so that the curvature radius on the lens surface 51b on the subject's eye side is larger than the curvature radius on the lens surface 51a on the light source side. It is confirmed that in a case where the curvature radius of the lens surface 51b on the subject's eye side becomes larger, an interval w between the lens surface 51b (however, the position closest to the subject's eye side in the lens surface 51b) and the pivot point Q is likely to be secured. In the example illustrated in Fig. 6, the lens surface 51b is a plane, and the curvature radius is infinite. Thus, it is easy to design the optical system which provides a sufficient interval w. Accordingly, as a result of forming the lens surface 51b by using the surface of the sufficiently large curvature radius, the operating distance is likely to be secured. For example, in this manner, an examiner easily carries out eyelid opening work of the subject (work for lifting an upper eyelid with a finger). According to one design solution of the optical system illustrated in Fig. 6 which is proposed by the inventor, it is possible to secure the interval w of approximately 13 mm while ensuring the imaging field angle such as a full angle of 100° or larger. If it is assumed that an examination window is shared by the lens surface 51b and the operating distance is a value obtained by subtracting approximately 3 mm, which is a typical interval from the corneal apex to the pupil of the human eye, from the interval w, it is possible to secure approximately 10 mm as the operating distance. The lens surface 51b may be a curved surface such as a spherical surface. In this case, for example, it is preferable that the curvature radius of the lens surface 51b is a value which is 10 times or greater than a desired operating distance.

The second lens system 50b is disposed between the first lens system 50a and the scanning unit 16 (more specifically, the first objective lens system 17). The second lens system 50b has at least one tilted lens. In a case where the second lens system 50b includes a plurality of lenses, two or more lenses may serve as the tilted lens. As illustrated in Fig. 6, all of the lenses may serve as the tilted lens.

The second lens system 50 illustrated in Fig. 6 includes a cemented lens 52 and convex meniscus lenses 53, 54, and 55, as the tilted lens. As illustrated in Fig. 6, each of the tilted lenses 52, 53, 54, and 55 is formed so that the lens surface on the light source side has the curvature radius larger than that of the lens surface 51a on the light source side in the first lens system (more specifically, the lens 51). Each of the tilted lenses may be a spherical lens. In addition, any of the tilted lenses 52, 53, 54, and 55 has positive power. However, the present invention is not necessarily limited thereto. The aspheric lens and/or the lens having negative power may be applied to some or all of the lenses included in the second lens system 50b. In the second objective lens system 50, similarly to the first objective lens system 17, the tilted lenses 52, 53, 54, and 55 are also disposed between the first lens system 50a and the scanning unit 16. In this manner, the reflected light on the lens surface of the first lens system 50a is restrained from being incident on the light receiving elements 25, 27, and 29.

The second lens system 50b may include the lens for correcting the aberration caused by the first lens system 50a. For example, the cemented lens 52 corrects the chromatic aberration. More specifically, the cemented lens 52 may be an achromatizing lens formed by joining at least two lenses having mutually different light dispersion characteristics (Abbe number) to each other. In order to restrain the laser light from being reflected on the joining surface of the cemented lens 52, in the cemented lens 52, the refractive indexes of the respective lenses and an adhesive for joining the respective lenses to each other may coincide with each other (including substantial coincidence) with each other. In addition, each lens included in the cemented lens 52 may be selected so that the power of the cemented lens 52 is zero.

All of the chromatic aberrations occurring in a state where the lens attachment 3 is mounted thereon do not need to be corrected by the cemented lens 52. The lens attachment 3 may further have a chromatic aberration correction lens 59 (achromatizing lens) which is not tilted. The chromatic aberration may be corrected using the chromatic aberration correction lens 59 together. In this case, it is preferable that the power of the lens 59 is zero (that is, total power of the concave lens and the convex lens is zero).

The convex meniscus lenses 53, 54, and 55 have positive power. Therefore, the positive power contributes to a configuration for bending the laser light to the pivot point Q. However, the bending amount is sufficiently smaller than that of the first lens system 50a.

In the present embodiment, the astigmatic difference (astigmatic difference at the pivot point P) caused by the optical scanners 16a and 16b is corrected (reduced) by the tilted lenses 172 and 173 disposed in the first objective lens system 17 disposed in the housing of SLO 1. Here, a case is conceivable where the astigmatic difference caused by the optical scanners 16a and 16b remains via the first the objective lens system 17. In a case where the laser light incident on the second objective lens system 50 has the above-described astigmatic difference, the tilted lens included in the second lens system 50b may be disposed to be tilted to the optical axis L1 in a direction so as to cancel (reduce) the astigmatic difference. For example, as illustrated in Fig. 6, the tilted lenses 52, 53, 54, and 55 are disposed to be tilted so that the tilted amount of the tilted lenses 52, 53, 54, and 55 in the second objective lens system 50 further reduces the remaining astigmatic difference.

In addition, as illustrated in Fig. 6, in a case where the second lens system 50b includes a plurality of tilted lenses, some of the tilted lenses and the remaining tilted lenses may be disposed in mutually reverse phases with respect to the optical axis so L3 (or the optical axis L1 of the irradiation optical system 10) of the lens attachment. As a specific example, in the second objective lens system 50b illustrated in Fig. 6, the tilted lenses 52 and 53 and the tilted lenses 54 and 55 are disposed to be tilted in mutually reverse phases with respect to the optical axis L3. In this manner, it is possible to cancel the tilt of the image plane caused by each of the tilted lenses 52 and 53 and the tilted lenses 54 and 55. The tilt of the image plane inside the second lens system 50b does not necessarily be corrected. For example, the tilt of the image plane caused by the tilted lens of the second lens system 50b may be corrected by disposing and tilting the lens included in the first lens system 50a. In this case, at least one lens included in the first lens system 50a is disposed in the reverse phase with at least one tilted lens included in the second lens system 50b with respect to the optical axis L3.

<Disposing Perforated Mirror>

In the above description, an example has been described in which the perforated mirror 13 is disposed at the position conjugate with the anterior ocular segment. In a case where the operating distance is properly set, in order to more preferably restrain the noise light, the perforated mirror 13 may be disposed to be located at a cornea conjugate position. In a case where SLO 1 has an aperture unit 230 as illustrated in Fig. 3 and one of apertures 232 and 233 for imaging the scattered light is disposed in the optical path of the light receiving optical system 20, the opening of the apertures 232 and 233 becomes large. Accordingly, a case is conceivable where the noise light cannot be sufficiently removed by the apertures 232 and 233. In particular, the noise light caused by corneal reflection is likely to be guided to the light receiving elements 25, 27, and 29. In contrast, in a case where the operating distance is properly set, the perforated mirror 13 is disposed so as to be located at the cornea conjugate position. In this manner, the corneal reflection is satisfactorily removed in the perforated mirror 13.

<Light Blocking Member>

The light blocking unit 22 is disposed at a position shifted from the fundus conjugate plane in the optical path between the perforated mirror 13 and the light receiving elements 25, 27, and 29. The light blocking unit 22 allows the light from the fundus conjugate plane to pass therethrough, and blocks at least a portion of the reflected light from the lens surface of the objective lens systems 17 and 50. In the present embodiment, the light blocking unit 22 blocks the light in the vicinity of the optical axis L2 of the light receiving optical system 20.

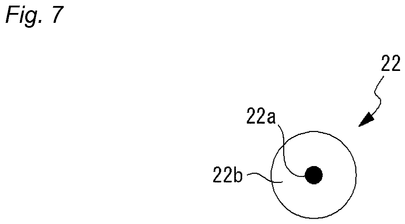

In the present embodiment, the light blocking unit 22 is a black spot plate having a black spot 22a forming the light blocking region and a light transmitting plate 22b having a ring-like opening formed therein (refer to Fig. 7). For example, the black spot 22a is disposed on the optical axis L2, and is disposed in order to block light in the vicinity of the optical axis L2. The light transmitting plate 22b is formed in a region away from the optical axis L2, and is disposed in order to transmit the light from the fundus conjugate plane. Herein, the vicinity of the optical axis L2 may be a region close to the optical axis L2 with respect to the outer edge of the passage region of the light from the fundus reflected on the perforated mirror 13. As will be described later, it is more preferable that the black spot 22a is formed in the region close to the optical axis L2 with respect to the inner edge of the passing region. A suitable setting range of the black spot 22a will be described later.

The installation position of the light blocking unit 22 (that is, the position shifted from the fundus conjugate plane) can be prescribed on the condition that the light blocking unit 22 is shifted from at least the substantially conjugate position of the fundus. That is, the light blocking unit 22 blocks the light (mainly including the light from the lens surface) in the vicinity of the optical axis L2 within the light directed to the light receiving elements 25, 27, and 29, and allows the light (mainly light from the fundus conjugate plane) in a region away from the optical axis L2 to pass therethrough. In this case, the light blocking unit 22 is configured to allow the light from the substantially conjugate plane (front and rear faces with respect to the focusing plane of the fundus) to pass therethrough.

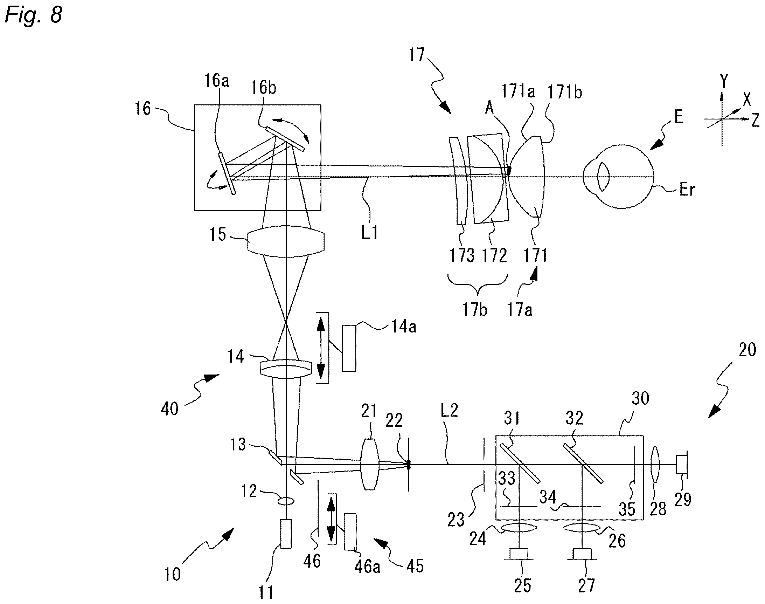

In addition, the installation position of the light blocking unit 22 may be located between the pupil conjugate position and the fundus conjugate position, which is shifted from both the pupil conjugate position and the fundus conjugate position. For example, as illustrated in Figs. 2 and 8, the installation position of the light blocking unit 22 may be located between the perforated mirror 13 and the pinhole plate 23. As described above, the present embodiment has a problem in that in the lenses 51 and 171 in which the bending amount of the laser light is relatively large, the light is reflected on the lens surface on the light source side. Therefore, the light blocking unit 22 may be disposed at the position conjugate with the lens surface 51a and 171a on the light source side of the lens 51 or the lens 171. For the sake of convenience, description will be made on the assumption that the light blocking unit 22 is disposed at the position conjugate with the lens surface 171a in Fig. 8. However, compared to the lens 171, in the lens 51 for more largely bending the laser light, the reflected light on the lens surface side on the light source side is likely to be guided to the light receiving elements 25, 27, and 29. Therefore, it is more preferable that the light blocking unit 22 is disposed at the conjugate position with the lens surface 51a on the light source side of the lens 51 (in this case, a ray diagram will be omitted). In addition, the light blocking units 22 may be disposed one by one for each of the conjugate position of the lens surface 171a and the conjugate position of the lens surface 51.

For example, the light blocking unit 22 removes the reflected light from the lens surfaces 51a and 171a by blocking the light in the vicinity of the optical axis of the light receiving optical system 20. An image of an irradiation region A of the laser light on the lens surfaces 51a and 171a is formed in a region B in the vicinity of the optical axis of the light receiving optical system 20 at the position of the light blocking unit 22. Here, the irradiation region A is successively displaced by the scanning of the laser light. However, the displacement of the reflected light from the lens surfaces 51a and 171a is canceled by the reflected light passing through the scanning unit 16. As a result, the image of the irradiation region A is formed in a prescribed region (specifically, a region in the vicinity of the optical axis L2) at the installation position of the light blocking unit 22 which is conjugate with the lens surface 171a or the lens surface 51a). That is, in a case where the reflected light from the lens surface 171a or the lens surface 51a is reflected on the perforated mirror 13, the reflected light is incident on the inside of the region B by a conjugate relationship. Therefore, the light blocking region using a black spot 22b is formed in the region B, thereby removing the reflected light from the lens surface 171a.

The present inventor confirms the following fact through simulation calculation using a ray tracing method. Even in a case where there are some errors between the installation position of the light blocking unit 22 and the conjugate position of the lens surface 171a or the lens surface 51a, the reflected light from the lens surface 171a or the lens surface 51a can be satisfactorily restrained by the light blocking unit 22. Accordingly, the installation positions of the light blocking unit 22 may be located away from each other in the front and rear of the conjugate position of the lens surface 171a or the lens surface 51a within a range which satisfies the object of the present disclosure. In a case where the diopter adjuster 40 adjusts the diopter of the optical system, deviation may occur between the light blocking unit 22 and the conjugate position of the lens surface 171a or the lens surface 51a. However, the deviation is allowable.

The light blocking region (black spot 22a) may be formed in a range where a viewing angle (apparent size) based on the pinhole 23a at the installation position of the light blocking unit 22 becomes equal to the viewing angle of an opening 13a of the perforated mirror 13.

For example, the light blocking region may be configured so that the viewing angle based on the pinhole 23a completely coincides with the opening 13a at the installation position of the light blocking unit 22. In this case, the reflected light from the lens surface 171a or the lens surface 51a is restrained from being incident on the light receiving elements 25, 27, and 29 while the light amount of the light from the fundus Er guided to the light receiving elements 25, 27, and 29 is restrained from decreasing. In addition, the light blocking region may be formed so that the viewing angle based on the pinhole 23a is larger than the viewing angle of the opening 13a within a range where the fundus reflected light is not completely blocked.

In the following description, an example will be described in which the light blocking unit 22a blocks the light having all wavelength ranges emitted from the laser light emitter 11. However, the light blocking unit 22a is not necessarily limited thereto. For example, the light blocking unit 22a may be a filter which transmits fluorescence (mainly a portion of the visible range) from the fundus and blocks observation light (mainly infrared light). In this case, excitation light and observation light are emitted from the laser light emitter 11 for irradiation. In this manner, it is possible to observe a fluorescent fundus image well while restraining the noise light in the observation image. SLO 1 may have a unit in which the light blocking member having the light blocking unit 22a formed by this filter and the light blocking member for blocking the light having all wavelength ranges emitted from the laser light emitter 11 are disposed for switching therebetween with respect to the optical axis L2.

<Polarizing Plate>

In addition, as illustrated in Fig. 2, in SLO 1, a polarizing unit 45 may be included in the light receiving optical system 20. The polarizing unit 45 has a polarizing plate 46 (deflector) and a drive mechanism 46a. Drive control of the drive mechanism 46a causes the polarizing plate 46 to be inserted into and removed from the optical path between the perforated mirror 13 and the light receiving elements 25, 27, and 29. The reflected light from the objective lens system 17 and the second objective lens system 51 may be blocked by a deflector plate 46. A direction of the linearly polarized light which can pass through the polarizing plate 46 may be adjusted so as to allow the light from the fundus to pass therethrough. A polarizing direction of the reflected light from the objective lens system 17 and the second objective lens system 51 is constant. In contrast, photoreceptor cells are anisotropic in the retina of the fundus. Accordingly, the fundus reflected light has a different polarizing direction from that of the reflected light from the objective lens system 17 and the second objective lens system 51. Therefore, the polarizing plate 46 is appropriately rotated (revolves) around the optical axis L2. In this manner, the deflector plate 46 can be adjusted so as to satisfy the above-described condition. For example, this adjustment work may be carried out at the time of product shipment.

<Another Embodiment relating to Objective Lens System>

As described above, in the present embodiment, the imaging field angle is switched by attaching or detaching the lens attachment 3. In other words, in the present embodiment, the fundus is imaged using the first imaging field angle via only the objective lens system 17 in the objective lens system 17 disposed in the main body 2 in advance and the second objective lens system 50 disposed in the lens attachment 3. In addition, the fundus is imaged using the second imaging field angle different from the first imaging field angle via both the objective lens system 17 and the second objective lens system 50. However, a configuration for switching the imaging field angle is not necessarily limited to the method according to the present embodiment. For example, the imaging field angle may be switched by switching a position relationship of the lenses in the objective lens system disposed in the main body of SLO. In this case, for example, the position relationship between the lenses included in the objective lens system is switched along the optical axis of the irradiation optical system. In this manner, the imaging field angle may be switched. In addition, the imaging field angle may be switched by exchanging the lens attachment mounted on the surface of the housing at the subject side of the main body. In this case, the main body of SLO may not necessarily have the objective lens system. At least some of the above-described characteristics with regard to the objective lens system 17 or the second objective lens system 50 in the present embodiment are applicable to any one of the cases. In this case, in any embodiment, at least a portion of the irradiation optical system 10 and at least a portion of the light receiving optical system 20 are housed in the same housing.

<Configuration of Control System>

Next, referring to Fig. 9, a control system of SLO 1 will be described. In SLO 1, each unit is controlled by a control unit 70. The control unit 70 is a processing device (processor) having an electronic circuit to perform control processing and calculation processing of each unit of SLO 1. The control unit 70 is realized by a central processing unit (CPU) and a memory. The control unit 70 is electrically connected to a storage unit 71 via a bus. In addition, the control unit 70 is electrically connected to each unit such as the laser light emitter 11, the light receiving elements 25, 27, and 29, the drive mechanisms 14a and 46a, the scanning unit 16, the input interface 75, and the monitor 80.

Various control programs and fixed data are stored in the storage unit 71. In addition, data may be temporarily stored in the storage unit 71. As illustrated in Fig. 9, the image captured by SLO 1 may be stored in the storage unit 71. However, the present invention is not limited thereto. The captured image may be stored in an external storage device (for example, a storage device connected to the control unit 70 via LAN and WAN).

For the sake of convenience, in the present embodiment, the control unit 70 also serves as an image processor. For example, based on the received signal output from the light receiving elements 25, 27, and 29, the control unit 70 forms the fundus image. In addition, the image processing (image processing and analysis) for the fundus image is also performed by the control unit 70. As a matter of course, the image processor may be a device separate from the control unit 70. As illustrated in Fig. 9, based on the signal from the respective light receiving elements 25, 27, and 29, the control unit 70 substantially simultaneously generates maximum three types of image based on each signal from the light receiving elements 25, 27, and 29.

In addition, the control unit 70 controls the above-described respective members, based on an operation signal output from the input interface 75 (operation input unit). The input interface 75 is an operation input unit for receiving an operation of an examiner. For example, the input interface 75 may be a mouse and a keyboard.

<Description of Operation>

The characteristics of the above-described optical system reduce possibilities that the reflected light from the objective lens system 17 and the second objective lens system 50 may be reflected on the fundus image. However, the restraining effect of reflection and the light amount of the light guided to the light receiving elements 25, 27, and 29 from the fundus Er is in a trade-off relationship. Accordingly, it is conceivable to provide the design solution and the imaging condition which allow the reflection of the reflected image from the objective lens system 17 and the second objective lens system 50. Therefore, in the present embodiment, the reflection of the reflected image is also reduced by the image processing.

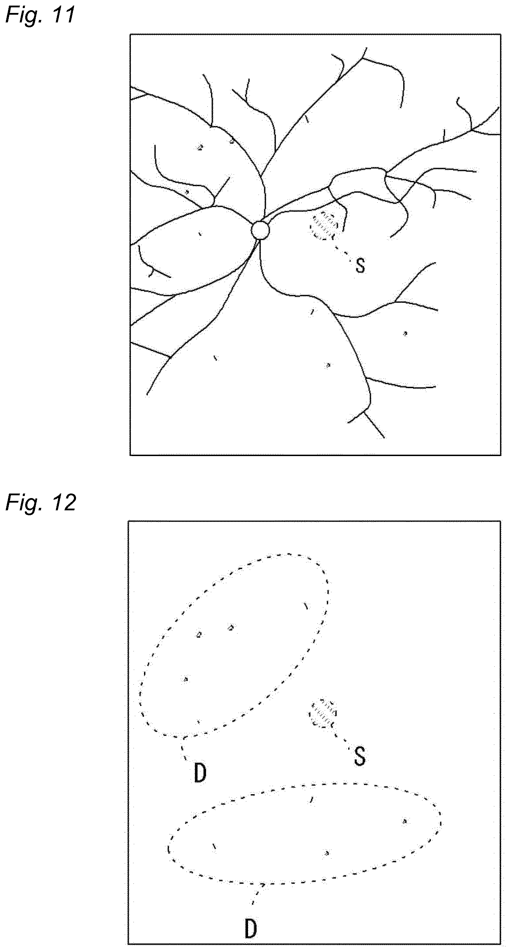

First, an overview of the processing performed in the control unit 70 according to the present disclosure will be described. The control unit 70 performs the imaging process on the fundus image, and images the fundus Er so as to acquire the fundus image as a captured image. In addition, the control unit 70 acquires a background image (also referred to as a "mask image") for the captured image (fundus image). The "background image" includes the reflected image generated by at least the objective lens system. The background image is obtained in such a way that the reflected image generated by at least the objective lens system is projected so that the subject's eye serving as the subject is not projected. In addition, the background image is utilized in contrast with the observation image in a background difference (fundus image in the present embodiment) which is one type of the image processing (refer to Fig. 12). The background image includes artifacts interfering with the observation and analysis of the fundus image, in addition the reflected image of the objective lens system.

This background image may be obtained based on the imaging performed by the photographic optical system in a "light blocked state". The "light blocked state" is realized by blocking the light on the subject's eye side of the lens which generates the reflected image in the objective lens system. In the objective lens system having a plurality of lenses, it is desirable to block the light at a position on the subject's eye side of the lens which generates a reflected image of an object. For example, the light may be blocked at a position of an optical column tip of the objective lens system. In this case, the reflected image caused by all of the lenses of the objective optical system can be included in the background image. The imaging timing of the background image may be before or after the imaging of the fundus image which is a target of the background difference (details will be described later).

The light may be blocked by disposing the "light blocking member" on the subject's eye side of the lens which generates the reflected image in the objective lens system. The light blocking member blocks the illumination light guided to the subject's eye by being disposed on the optical path of the illumination light. The light blocking member may be a member separate from the device body or may be provided in advance for the device body. Specifically, a lens cap, a shutter, or a blackout can be employed as the light blocking member. In a case of the shutter, a drive mechanism (shutter mechanism) for inserting the shutter (light blocking member) into or removing the shutter from the optical path of the illumination light may be disposed in the device body or the optical column (for example, the optical column of the lens attachment may also be employed) of the objective lens system. The shutter mechanism is switched between a closed state where the light on the subject's eye side of the lens which generates the reflected image is blocked by the light blocking member in response to insertion and removal of the light blocking member and an opened state where the light on the subject's eye side of the lens which generates the reflected image is not blocked by the light blocking member. The shutter mechanism may be a mechanism for switching a state the optical column tip of the objective lens system is closed by a lid of the light blocking member and a state where the lid is detached therefrom. In the light blocking member, it is preferable that the surface irradiated with the illumination light is formed of a material having a high absorption rate of the illumination light. For example, black flocked paper may be used. In addition, for example, a new material using carbon nanotubes has attracted attention in recent years, and this new material may be employed.

In addition, the light blocked state caused by the light blocking member may be detected by a sensor. Then, the background image may be captured (acquired) based on a detection result of the light blocked state. For example, in a case where the shutter mechanism is provided, the background image may be captured based on switching of the shutter mechanism from the opened state to the closed state.

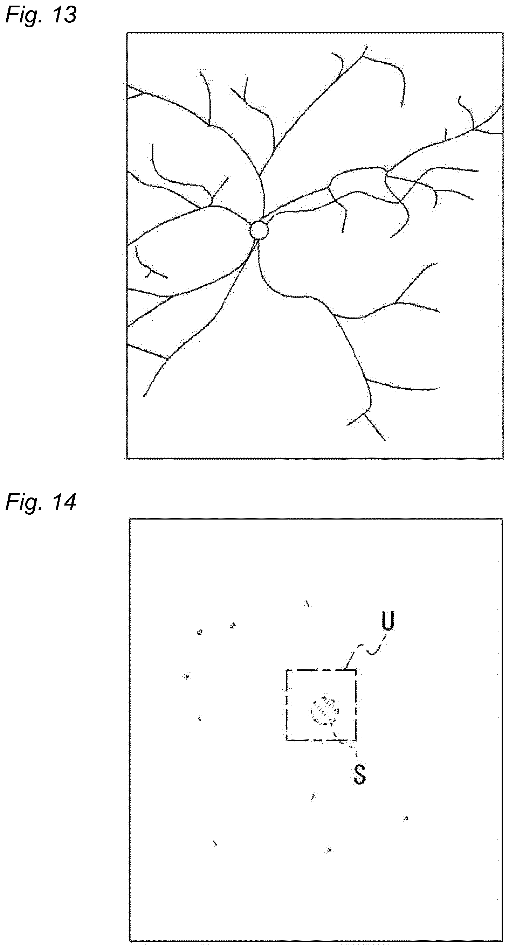

In the present embodiment, the background difference based on the fundus image (refer to Fig. 11) and the background image (refer to Fig. 12) is obtained by the control unit 70. In this manner, in the fundus image, an image in which the influence of the reflected image by the objective lens system is restrained is generated as a difference image (refer to Fig. 13). In other words, reflection of the reflected image on the fundus image by the objective lens system is posteriorly reduced by the image processing. The difference image in the present embodiment may be generated by directly obtaining the difference between the fundus image and the background image. In addition, the difference image may be generated by correcting at least one of the fundus image and the background image and obtaining the difference with regard to the corrected image.

Here, there is a possibility that in the difference image obtained as a result from the background difference, random noise may increase compared to the original fundus image. Therefore, for example, the control unit 70 may acquire the difference image for each of a plurality of fundus images obtained by imaging the same region at different timing, and may generate an averaged image by using each difference image. As a result, it is possible to obtain the fundus front image whose random noise is restrained together with the reflected image generated by the objective lens system.

In addition, for example, a case is conceivable where a resonant scanner is applied to the scanning unit of the irradiation optical system. In some cases, laser light scanning performed by the resonant scanner is unstable due to disturbance. Therefore, there is a possibility that the magnification of the scanning direction in at least a portion of the image may be different between the fundus image and the background image. As a result, it is conceivable that an appearance position of the artifact is shifted between the fundus image and the background image. If a position of a minute artifact caused by dust adhering to the optical system of SLO 1 is shifted between the fundus image and the background image, the background difference is obtained for the minute artifact. Instead, the minute artifact may increase.

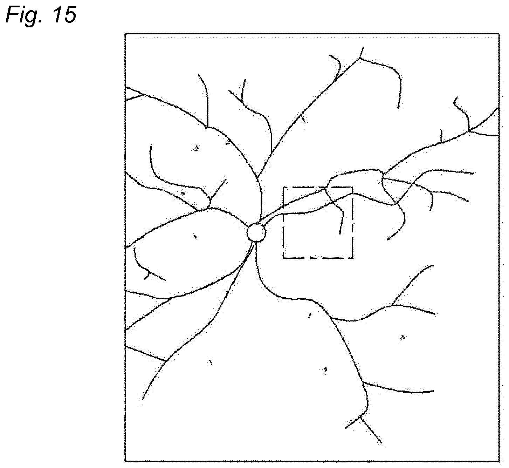

In contrast, the control unit 70 may acquire the difference image by extracting a portion including the reflected image generated by the objective lens system from the background image and obtaining the background difference based on the extracted image (for the sake of convenience, referred to as a "partial background image, for example, a region U in Fig. 14) and the fundus image. That is, the difference image is generated by subtracting a luminance value in the partial background image for each pixel from a region corresponding to the partial background image in the fundus image. As a result, the minute artifact is minimized to the same extent as the original fundus image, and the front image of the fundus whose influence by the objective lens system is restrained is generated as the difference image (for example, refer to Fig. 15). In addition, a processing period of time in the image processing can be shortened compared to a case where the background difference is obtained using the whole background image.

Here, in the fundus image and the background image, a position of the region of the reflected image generated by the objective lens system is substantially constant. For example, the reflected image is generated in an image center. Therefore, the partial background image may be obtained by extracting a region at a predetermined position in the background image. In addition, the control unit 70 may detect the region of the reflected image generated by the objective lens system from the background image, and may extract the partial background image from the detected region.

Incidentally, in order to satisfactorily reduce the reflected image generated by the objective lens system, it is preferable that the imaging conditions of the fundus image and the background image are the same as each other. Alternatively, it is preferable to restrain the influence caused by the difference in the imaging conditions by correcting one image or both images of the fundus image and the background image. The imaging conditions include a condition (for example, a set value) on at least one of the output of the illumination light, the gain of the received light signal, and the diopter in the photographic optical system.

For example, in order to allow the imaging conditions to coincide with each other, the control unit 70 may capture the background image without changing (without accepting) the imaging condition after capturing the fundus image which is a target of the background difference. In this case, the fundus image and the background image are acquired in a series of imaging operations.

In addition, the control unit 70 may cause the storage unit to store information by associating information indicating the imaging condition when the fundus image is captured with the fundus image. Then, the control unit 70 may capture the background image by reproducing the imaging condition stored in the storage unit. In this case, even if the imaging condition is reset or changed once after the fundus image is captured, it is possible to obtain the satisfactory background image by obtaining the background difference with the fundus image.

In addition, while switching the imaging condition relating to at least one of the output of the illumination light, the gain of the received light signal, and the diopter of the photographic optical system, the control unit 70 may acquire in advance a plurality of the background images whose imaging conditions are different from each other by repeatedly capturing the background images in a light blocked state. In this case, the background image serving as the background difference with the fundus image is selected from a plurality of the background images which are acquired in advance. For example, the control unit 70 may select the background image acquired on the imaging condition which is the same as or close to the imaging condition of the fundus image. The background difference between the selected background image and the fundus image is obtained, thereby obtaining the difference image. In this case, the difference image in which the reflected image generated by the objective lens system is satisfactorily restrained can be obtained immediately after the fundus image is captured (for example, with a slight time lag from when the fundus image is captured). For example, according to this method, real-time moving image using the difference image can be displayed by sequentially acquiring the observation images of the fundus image and by sequentially generating and displaying the difference images based on the respective fundus images.

Here, in some cases, the output characteristics of the light source and the gain characteristics of the light receiving element may be successively and slowly changed. It is preferable to appropriately update the background image. For example, the background image may be periodically updated every few days (including every few weeks and every few months). In addition, an update time limit may be managed. In a case where the predetermined update time approaches, the control unit 70 may cause a monitor to display guide information for notifying the update of the background image.

Next, an example of a method for correcting one or both of the fundus image and the background image will be described. For example, the control unit 70 may acquire in advance at least one background image when the imaging condition relating to at least one of the output of the illumination light and the gain of the received light signal is a first imaging condition. Then, in a case where an imaging condition (second imaging condition) in the fundus image serving as a target of the background difference is different from the first imaging condition in the background image, the control unit 70 may correct contrast or brightness in at least one of the fundus image and the background image, and may obtain the background difference, based on the corrected image. In this case, the correction amount in image correction (that is, alignment) relating to the contrast or the brightness may be acquired in accordance with information indicating the first imaging condition and information indicating the second imaging condition. For example, the correction value may be acquired by a calculation result using a predetermined computation expression whose function is the information indicating the first imaging condition and the information indicating the second imaging condition. In addition, the correction value may be obtained by referring to a look-up table whose correction value is associated with each combination of the information indicating the first imaging condition and the information indicating the second imaging condition. For example, the correction amount of the contrast and the brightness for correcting the background image on the first imaging condition to the background image on the second imaging condition may be obtained from a difference between the background image on the first imaging condition and the background image on the second imaging condition. In this case, for example, the correction value stored in the look-up table can be obtained based on a plurality of the background images having different imaging conditions. According to this method, many background images may not be stored in advance in the storage unit. In addition, according to this method, it becomes possible to obtain the satisfactory difference image immediately after the fundus image is captured. Therefore, for example, it is also possible to display the real-time moving image by using the difference image.

In this method, as the background image acquired in advance, a plurality of images may be used in which the imaging conditions (second imaging condition) relating to at least one of the output of the illumination light and the gain of the received light signal are different from each other. The background image captured on the second imaging condition closer to the imaging condition (first imaging condition) of the fundus image is selected from a plurality of the background images captured on mutually different imaging conditions. Then, the contrast or the brightness may be corrected in at least one of the fundus image and the background image by using the correction value in accordance with the information showing the first imaging condition and the information showing the second imaging condition, and the background difference may be obtained based on the corrected image. In this case, the correction value may be used to interpolate the difference in the imaging conditions between a plurality of the background images. As a result, it is possible to restrain the number of the background images acquired in advance. In addition, there are time-dependent changes in the output characteristics of the light source and the gain characteristics of the light receiving element. Accordingly, the correction value and the background image may be appropriately updated.

The background difference can also be applied to a case where at least two images are synthesized between a fundus image (referred to as a "first fundus image") captured using the fundus reflected light of first illumination light and a fundus image (referred to as a "second fundus image") captured using the fundus reflected light of second illumination light (which has a different wavelength range from that of the first illumination light). The "synthesis" may be a process of generating one image by mixing colors corresponding to the respective images for each corresponding pixel in at least two images to generate one image.

For example, a background image (referred to as a "first background image") in the first wavelength range and a background image (referred to as a "second background image") in the second wavelength range may be acquired. Then, the background difference between the first fundus image and the first background image and the background difference between the second fundus image and the second background image may be respectively obtained so as to generate the first difference image and the second difference. The first difference image and the second difference may be synthesized so as to obtain a synthesized difference image. It is conceivable that the reflected image in the objective lens system appears in the different intensity distribution and range depending on each wavelength range. Therefore, the background difference is obtained for each fundus image in each wavelength range, thereby suitably restraining the reflected image of the objective lens system in the first difference image and second difference image. Since these images are synthesized with each other, a satisfactorily synthesized difference image is obtained.

However, the background difference does not necessarily need to be obtained for each fundus image in each wavelength range. For example, the synthesized difference image may be obtained using the background difference based on the first synthesized image of the first fundus image and the second fundus image, and the second synthesized image between the first background image and the second background image. In addition, the difference image may be generated using the fundus image and the background image which have mutually different wavelength ranges of the illumination light. A synthetic image using the difference images in that way may be generated.

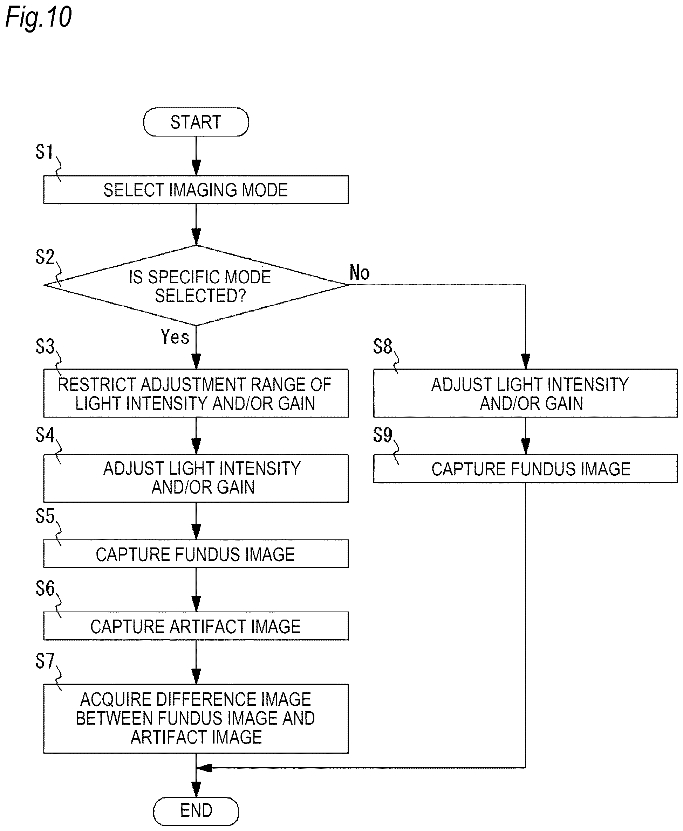

Next, a specific operation in SLO 1 will be described with reference to a flowchart illustrated in Fig. 10. After alignment (S1) between the subject's eye and the device is completed, an imaging mode is selected by the control unit 70 (S2). For example, a plurality of imaging modes corresponding to the imaging method and the imaging field angle may be prepared in advance. For example, in the present embodiment, the imaging mode is largely classified into a "normal mode" serving as the imaging mode in a state where the lens attachment 3 is not mounted thereon, and a "wide angle mode" serving as the imaging mode in a state where the lens attachment 3 is mounted thereon. That is, in the "wide angle mode", the imaging field angle is widened compared to the "normal mode". In each of the "normal mode" and the "wide angle mode", as the imaging mode, the following modes may be prepared such as a mode for capturing the fundus image by using the fundus reflected light (more specifically, an "IR mode" for infrared imaging and a "color mode" for color imaging), a mode for capturing the fundus image by using fluorescence emitted from the fundus (more specifically an "ICG mode" for ICG imaging, an "FA mode" for FAG imaging, and an "FAF mode" for autofluorescence imaging). For example, these imaging modes may be selected in accordance with a selection operation of the imaging mode through the input interface 75. In addition, the control unit 70 may automatically select the imaging mode based on a detection result of the device state and/or in accordance with a predetermined imaging sequence. In this case, for example, in response to a signal from the sensor 8 (refer to Fig. 1) indicating whether or not the lens attachment 3 is mounted thereon, the control unit 70 may select between the "normal mode" and the "wide angle mode".

In accordance with the selected imaging mode, the control unit 70 switches the settings of the photographic optical system (the irradiation optical system 10 and the light receiving optical system 20), such as the wavelength range of the laser light from at least the laser light emitter 11 and the light receiving element used for observing and capturing the fundus image among the three light receiving elements 25, 27, and 29 (S3).

Next, the control unit 70 determines whether or not a specific imaging mode is selected (S4). The specific imaging mode described herein means an imaging mode using the fundus reflected light. For example, the "IR mode" and the "color mode" are included therein. In the present embodiment, in a case of the fluorescence imaging, due to a filter (for example, dichroic mirrors 31 and 32. Alternatively, a filter (not illustrated) may be employed) disposed in the light separator 30, the light in the wavelength (that is, the wavelength range the reflected light from the objective lens system 17 and the second objective lens system 50) of excitation light is less likely to be incident on the light receiving element for obtaining a fluorescent image. Therefore, the above-described reflection is less likely to cause a problem in the captured image. However, in some cases, the fluorescent image may also have the reflected image generated due to the reflection on the objective lens system 17 and the second objective lens system 50. Therefore, the background difference may also be obtained for the fluorescent image. In this case, it is preferable to obtain the background difference with the background image captured on the same imaging condition as that when the fluorescence imaging is performed on the fundus. For example, in a configuration in which an exciter filter and a barrier filter are inserted into the optical path of the photographic optical system during the fluorescence imaging, the background image may be acquired through the imaging on the condition that filters are similarly disposed therein. In addition, for example, in a case where the above-described reflection causes a problem in only the "wide angle mode" in the "normal mode" and the "wide angle mode", "the normal mode" and "the wide angle mode", the "wide angle mode" may be regarded as a condition of the specific imaging mode.

In a case of the specific imaging mode (S4: Yes), the control unit 70 restricts at least one adjustment range of the light amount and the gain (S5). For example, in the fundus image and the background image (to be described later), the adjustment range is restricted to a range where a luminance value of a region (for example, a central portion of the fundus image) in which the reflected image is reflected due to the objective lens system 17 and the second objective lens system 50 is not saturated (saturation). The reason is that information indicating the structure of the fundus is not included in the region in a case where the luminance value is saturated. For example, this adjustment range may be set in advance based on a result of experiment, or may be set based on the observation image. The "restriction on the adjustment range" means that the adjustment range is narrower than that in the imaging mode other than the specific imaging mode in the imaging modes of SLO 1. The "restriction on the adjustment range" includes that at least one of the light quantity and the gain is switched to a certain value (fixed value).

Next, the fundus image is captured (S6). For example, in a case of the "color mode", a color fundus image is captured. The light of red, green and blue wavelength ranges can be simultaneously emitted from the laser light emitter 11 (may be emitted at different timing and sequentially). Based on the signal from the respective light receiving elements 25, 27, and 29, the reflected image based on the light of the red, green and blue wavelength ranges is formed. Then, the color fundus image is formed by synthesizing these reflected images (refer to Fig. 11). The color fundus image obtained in this way may be stored in the storage unit 71.

Next, in the present embodiment, the background image is captured (S7). The background image includes artifacts interfering with the observation and analysis in the fundus image. In the present embodiment, a typical artifact is a reflected image S caused by the objective lens systems 17a and 51a. In addition, images of dust adhering to the optical system, a scratch, dirt, and flares of the optical system, can also be included in the background image as the artifact. In addition, in SLO, it is conceivable that the reflection of the edge of the optical scanner such as the galvano mirror also occurs as the artifact. As an example, Fig. 12 illustrates an image D of the dust.

For example, the background image may be an SLO image captured by blocking the light at the subject's eye E and the position of the lens surface (for example, the lens surface 51a and the lens surface 171a) disposed closest to the subject's eye E. The light blocking" may be realized by disposing the light blocking member between the lens surface 51a (alternatively, the lens surface 171a) closest to the subject's eye E side and the subject's eye E so that the fundus is not reflected on the SLO image captured as the background image. The light blocking member does not necessarily have to be installed in advance in the device. For example, a lens cap, a shutter, and a blackout can be employed as the light blocking member. A shutter mechanism may be disposed in the main body of SLO 1 or in the lens attachment 3. In this case, the shutter mechanism blocks the light by pulling down the shutter at the rear stage (side away from the light source) of the lens 51 and the lens 171.

In addition, it is not always necessary to realize the light blocking by disposing the light blocking member at the position of the lens surface 51a (alternatively, the lens surface 171a). For example, the fundus Er is not reflected on the SLO image by closing the eyelid of the subject's eye E. Therefore, it is also conceivable that light blocking is performed by closing the eyelid of the subject. In this case, for example, the control unit 70 may detect blinking of the subject's eye and may capture the background image while the eyelid is closed by the blinking.

The background image may be captured by detecting that the light is blocked and by regarding the detection as a trigger (in other words, based on a detection signal). Specifically, for example, a sensor for detecting whether the light is blocked at the position of the lens surface closest to the subject's eye side may be disposed in the lens attachment 3 and/or the main body of SLO 1. Based on the signal from that sensor, the control unit 70 may identify whether or not the light is blocked. In addition, if blinking is employed, an anterior ocular segment camera (not illustrated) may detect the blinking based on a result of imaging the anterior ocular segment. Alternatively, the blinking may be detected based on a histogram of the observation image. However, in a case where a light blocked state is detected, the background image may not be automatically captured. For example, in a case where an examiner confirms that the light is blocked and inputs a predetermined operation to the input interface 75, the background image may be captured based on the input.

In a process of S7, after the fundus image is captured in S6, the output of the light source (light amount), the gain of the light receiving elements 25, 27, and 29, and the imaging condition relating to the diopter of the optical system are not changed from when the fundus image is acquired in S6, and the imaging is performed in a state where the light is blocked. As a result of this imaging, the background image is acquired.

Next, the control unit 70 acquires the difference image caused by the background difference based on the fundus image and the background image (S8). In the difference image, the influence of the artifact on the fundus image is restrained. For example, as illustrated in Fig. 13, an image where the reflected image S caused by the objective lens systems 17a and 51a and the image D of the dust are removed from the fundus image illustrated in Fig. 11 is acquired as the difference image. The acquired difference image may be stored in the storage unit 71. Alternatively, the acquired difference image may be displayed on the monitor 75. In this way, the reflection of the reflected image which is caused by the objective lens system in the fundus image is posteriorly reduced by the image processing.

In a processing of S8, contrast adjustment of the difference image may be performed in conjunction with the background difference. For example, a correction process for adjusting the contrast may be included in the process of S8 so that a histogram of an intermediate image (one type of the difference image) obtained using the background difference is deployed in a predetermined gradation range.

Here, the difference image after the contrast adjustment is represented by a first gradation number (for example, 8 bits). The first gradation number may be a gradation number when the difference image is displayed on the monitor 75. In contrast, SLO 1 may acquire an image represented by a second gradation number (for example, 12 bits) greater than the first gradation number, by performing the processes of S6 and S7. In other words, both the fundus image and the background image which serve as a target of the background difference may be represented by the second gradation number. Then, the control unit 70 obtains the background difference between the images represented by the second gradation number, and acquires the intermediate image (one type of the difference image). Then, after the contrast adjustment is performed on the intermediate image, the image obtained after the contrast adjustment may be compressed so as to acquire the difference image represented by the first gradation number. In this way, the artifact can be more accurately removed.

In the present embodiment, in order to obtain the difference image, the background difference is obtained based on the background image and the fundus image the background difference which have mutually the same imaging condition (that is, the imaging condition relating to the output of the light source, the gain of the light receiving elements 25, 27, and 29, and the diopter of the optical system). Since the imaging conditions are the same as each other, a difference is less likely to occur between the artifact components reflected on each of the background image and the fundus image. Therefore, the influence of the artifact on the difference image is likely to be restrained.

The image processing of the above-described background difference can be appropriately applied to SLO having an ocular refractive system (lens system) in the objective optical system. However, as described above, if the luminance value of the reflected image S is saturated in the fundus image, as a result of removing the artifact by using the background difference, it is conceivable that the information of the fundus cannot be obtained at the position of the reflected image S in the difference image. Therefore, in order to restrain the luminance value from being saturated in the reflected image S, at least one of the characteristics for restraining the reflection of the reflected light from the objective lens system 17 and the second objective lens system 50 described in the present embodiment may be appropriately used in conjunction with the image processing.

In addition, the image processing of the above-described background difference is not limitedly applied to SLO. In addition to SLO, the image processing may be applied to "an ophthalmologic imaging device that includes photographic optical system including the objective lens system that includes at least one lens, the irradiation optical system which irradiates the subject's eye with the illumination light emitted from the light source via the objective lens system, and the light receiving optical system including the light receiving element which receives the reflected light of the illumination light on the subject's eye via the objective lens system, and that generates the captured image based on the signal output from the light receiving element". This ophthalmologic imaging device may be a fundus imaging device. In this case, the irradiation optical system irradiates the fundus of the subject's eye with the illumination light, and the light receiving optical system causes the light receiving element to receive the fundus reflected light of the illumination light. For example, as the fundus imaging device other than SLO, a fundus camera is conceivable. In addition, the ophthalmologic imaging device may be an anterior ocular segment camera.

Hitherto, the description has been made with reference to the embodiment. However, the contents of the embodiment can be appropriately modified in embodying the present disclosure.

[Description of Reference Numerals and Signs]

1 SLO
3 LENS ATTACHMENT
10 IRRADIATION OPTICAL SYSTEM
11 LASER LIGHT EMITTER
16 SCANNING UNIT
16a, 16b OPTICAL SCANNER
17 OBJECTIVE LENS SYSTEM
20 LIGHT RECEIVING OPTICAL SYSTEM
25, 27, 29 LIGHT RECEIVING ELEMENT
50 SECOND OBJECTIVE LENS SYSTEM
51, 171 ASPHERIC LENS
52, 172 CEMENTED LENS
171a, 172a, 172b LENS SURFACE
Er FUNDUS
L1,L3 OPTICAL AXIS
P,Q PIVOT POINT

## Claims

1. A scanning laser ophthalmoscope (1) comprising:

   an irradiation optical system (10) that includes a scanning unit (16) for scanning a fundus of a subject's eye with laser light emitted from a light source (11), and an objective lens system (17) that is disposed between the subject's eye and the scanning unit so as to form a pivot point around which the laser light guided from the scanning unit pivots in response to an operation of the scanning unit; and
   a light receiving optical system (20) that shares the objective lens system with the irradiation optical system, and that has a light receiving element (25,27,29) for receiving light from the fundus irradiated with the laser light, wherein the objective lens system includes a first lens system (17a) for bending the laser light toward the pivot point and a second lens system (17b) disposed between the first lens system and the scanning unit, and **characterised in that** the second lens system has a tilted lens (172, 173) disposed to be tilted to an optical axis of the irradiation optical system, and a lens surface (172a, 173a) on the light source side in the tilted lens has a curvature radius larger than that of a lens surface on the light source side in the first lens system.

2. The scanning laser ophthalmoscope according to claim 1, wherein the second lens system corrects an aberration caused by the first lens system.

3. The scanning laser ophthalmoscope according to claim 2, wherein
   the light source emits at least the laser light having a first wavelength and laser light having a second wavelength different from the first wavelength, and
   the second lens system includes a cemented lens which corrects a chromatic aberration caused by the first lens system with regard to at least the laser light having the first wavelength and the laser light having the second wavelength.

4. The scanning laser ophthalmoscope according to claim 3, wherein refractive indexes of a plurality of lenses further included in the cemented lens and an adhesive for joining the plurality of lenses to each other have the same value.

5. The scanning laser ophthalmoscope according to any one of claims 1 to 4, wherein the first lens system is disposed so that an optical axis of the first lens system extends along an optical axis of the irradiation optical system.

6. The scanning laser ophthalmoscope according to any one of claims 1 to 5, wherein the first lens system includes at least one lens which is disposed to be tilted to the optical axis of the irradiation optical system in a direction to cancel a tilt of an image plane on a fundus conjugate plane which is caused by the tilted lens.

7. The scanning laser ophthalmoscope according to any one of claims 1 to 6,
   wherein the first lens system is an aspheric lens which restrains a spherical aberration of image formation at the pivot point by forming at least one lens surface thereof in an aspheric shape.

8. The scanning laser ophthalmoscope according to any one of claims 1 to 7,
   wherein the second lens system is disposed between an intermediate image plane formed closest to the first lens system within an intermediate image plane of the fundus which is formed using the objective lens system, and the first lens system.

9. The scanning laser ophthalmoscope according to any one of claims 1 to 8, wherein
   the scanning unit has two optical scanners which are disposed at mutually different positions and which have mutually different scanning directions of the laser light, and
   the tilted lens is disposed to be tilted to the optical axis of the irradiation optical system in a direction to cancel an astigmatic difference at the pivot point which is caused by the two optical scanners.

10. The scanning laser ophthalmoscope according to any one of claims 1 to 9, further comprising:

    a housing that houses at least a portion of the irradiation optical system and at least a portion of the light receiving optical system; and
    a lens attachment mounted on a surface of the housing at a subject side,
    wherein the objective lens system is disposed in the lens attachment.

11. The scanning laser ophthalmoscope according to any one of claims 1 to 9, further comprising:

    a housing that houses at least a portion of the irradiation optical system and at least a portion of the light receiving optical system; and
    a lens attachment that switches an imaging field angle in accordance with attachment to or detachment from a surface of the housing at a subject side, and that has a second objective lens system for bending the laser light incident via the objective lens system toward a second pivot point different from the pivot point,
    wherein the second objective lens system includes at least one second tilted lens disposed to be tilted to the optical axis.

12. The scanning laser ophthalmoscope according to claim 11,
    wherein the scanning unit has two optical scanners which are disposed at mutually different positions and which have mutually different scanning directions of the laser light, and
    wherein the second tilted lens is disposed to be tilted to the optical axis of the irradiation optical system or the second objective lens system to cancel an astigmatic difference remaining at the second pivot point.

13. The scanning laser ophthalmoscope according to claim 11 or 12,
    wherein the lens attachment has at least two tilted lenses disposed in mutually reverse phases with respect to the optical axis so as to cancel a tilt of an image plane on a fundus conjugate plane.

14. A scanning laser ophthalmoscope comprising:

    an irradiation optical system including a scanning unit for scanning a fundus of a subject's eye with laser light emitted from a light source, and an objective lens system that is disposed between the subject's eye and the scanning unit so as to form a pivot point around which the laser light guided from the scanning unit pivots in response to an operation of the scanning unit; and
    a light receiving optical system that shares the objective lens system with the irradiation optical system, and that has a light receiving element for receiving light from the fundus irradiated with the laser light,
    wherein the scanning unit has two optical scanners which are disposed at mutually different positions and which have mutually different scanning directions of the laser light,
    wherein the objective lens system includes a first lens system for bending the laser light toward the pivot point and a second lens system disposed between the first lens system and the scanning unit, and **characterised in that** the second lens system has a tilted lens disposed to be tilted to an optical axis of the irradiation optical system, and the tilted lens is disposed to be tilted to the optical axis of the irradiation optical system to cancel an astigmatic difference at the pivot point which is caused by the two optical scanners.

**Patentansprüche**

1. Laser-Abtastophthalmoskop (1) umfassend;
   ein optisches Bestrahlungssystem (10), das eine Abtasteinheit (16) zum Abtasten eines Augenhintergrunds eines Auges einer Person mit von einer Lichtquelle (11) emittiertem Laserlicht und ein Objektivlinsensystem (17) umfasst, das zwischen dem Auge der Person und der Abtasteinheit zum Bilden eines Drehpunkts angeordnet ist, um den herum sich das von der Abtasteinheit geführte Laserlicht als Reaktion auf eine Betätigung der Abtasteinheit dreht; und ein optisches Lichtaufnahmesystem (20), welches das Objektivlinsensystem mit dem optischen Bestrahlungssystem gemeinsam verwendet, und das ein Lichtaufnahmeelement (25, 27, 29) zum Aufnehmen von Licht vom mit dem Laserlicht bestrahlten Augenhintergrund aufweist,
   wobei das Objektivlinsensystem ein erstes Linsensystem (17a) zum Beugen des Laserlichts zum Drehpunkt hin und ein zweites Linsensystem (17b) umfasst, das zwischen dem ersten Linsensystem und der Abtasteinheit angeordnet ist, und
   **dadurch gekennzeichnet ist, dass** das zweite Linsensystem eine geneigte Linse (172, 173) aufweist, die zu einer optischen Achse des optischen Bestrahlungssystems geneigt angeordnet ist, und eine Linsenoberfläche (172a, 173a) auf der Lichtquellenseite in der geneigten Linse einen größeren Krümmungsradius als den einer Linsenoberfläche auf der Lichtquellenseite im ersten Linsensystem aufweist.

2. Laser-Abtastophthalmoskop nach Anspruch 1, wobei das zweite Linsensystem eine durch das erste Linsensystem verursachte Aberration korrigiert.

3. Laser-Abtastophthalmoskop nach Anspruch 2, wobei
   die Lichtquelle zumindest das Laserlicht mit einer ersten Wellenlänge und das Laserlicht mit einer von der ersten Wellenlänge abweichenden zweiten Wellenlänge emittiert, und
   das zweite Linsensystem eine Kittlinse umfasst, die eine durch das erste Linsensystem verursachte chromatische Aberration in Bezug auf zumindest das Laserlicht mit der ersten Wellenlänge und das Laserlicht mit der zweiten Wellenlänge korrigiert.

4. Laser-Abtastophthalmoskop nach Anspruch 3, wobei die Brechungsindizes einer Vielzahl von Linsen, die zudem in der Kittlinse enthalten sind, und eines Klebstoffs zum Verbinden der Vielzahl von Linsen miteinander den gleichen Wert aufweisen.

5. Laser-Abtastophthalmoskop nach einem der Ansprüche 1 bis 4, wobei das erste Linsensystem so angeordnet ist, dass sich eine optische Achse des ersten Linsensystems entlang einer optischen Achse des optischen Bestrahlungssystems erstreckt.

6. Laser-Abtastophthalmoskop nach einem der Ansprüche 1 bis 5, wobei das erste Linsensystem zumindest eine Linse enthält, die so angeordnet ist, dass diese zur optischen Achse des optischen Bestrahlungssystems in einer Richtung zum Aufheben einer Neigung einer Bildebene auf einer Augenhintergrund-Konjugatebene geneigt ist, die durch die geneigte Linse verursacht wird.

7. Laser-Abtastophthalmoskop nach einem der Ansprüche 1 bis 6, wobei das erste Linsensystem eine asphärische Linse ist, die eine sphärische Aberration der Bilderzeugung am Drehpunkt durch Bilden zumindest einer Linsenoberfläche davon in asphärischer Form einschränkt.

8. Laser-Abtastophthalmoskop nach einem der Ansprüche 1 bis 7, wobei das zweite Linsensystem zwischen einer Zwischenbildebene, die zum ersten Linsensystem innerhalb einer Zwischenbildebene des Augenhintergrunds am nächsten ausgebildet ist, die unter Verwendung des Objektivlinsensystems gebildet ist, und dem ersten Linsensystem angeordnet ist.

9. Laser-Abtastophthalmoskop nach einem der Ansprüche 1 bis 8, wobei
   die Abtasteinheit zwei optische Scanner aufweist, die an voneinander unterschiedlichen Positionen angeordnet sind und die voneinander unterschiedliche Abtastrichtungen des Laserlichts aufweisen, und
   die geneigte Linse zur optischen Achse des optischen Bestrahlungssystems in einer Richtung zum Aufheben einer durch die zwei optischen Scanner verursachten astigmatischen Abweichung am Drehpunkt geneigt angeordnet ist.

10. Laser-Abtastophthalmoskop nach einem der Ansprüche 1 bis 9, ferner umfassend:

ein Gehäuse, in dem zumindest ein Bereich des optischen Bestrahlungssystems und zumindest ein Bereich des optischen Lichtaufnahmesystems untergebracht sind; und
eine Linsenbefestigung, die an einer Oberfläche des Gehäuses an einer Objektseite angebracht ist, wobei das Objektivlinsensystem in der Linsenbefestigung angeordnet ist.

11. Laser-Abtastophthalmoskop nach einem der Ansprüche 1 bis 9, ferner umfassend:

ein Gehäuse, in dem zumindest ein Bereich des optischen Bestrahlungssystems und zumindest ein Bereich des optischen Lichtaufnahmesystems untergebracht sind; und
eine Linsenbefestigung, die einen Bildgebungsfeldwinkel gemäß der Befestigung an oder der Entfernung von einer Oberfläche des Gehäuses an einer Objektseite umschaltet, und ein zweites Objektivlinsensystem zum Beugen des über das Objektivlinsensystem einfallenden Laserlichts in Richtung eines zweiten Drehpunkts aufweist, der sich vom Drehpunkt unterscheidet,
wobei das zweite Objektivlinsensystem zumindest eine zweite geneigte Linse umfasst, die zur optischen Achse geneigt angeordnet ist.

12. Laser-Abtastophthalmoskop nach Anspruch 11, wobei die Abtasteinheit zwei optische Scanner aufweist, die an voneinander unterschiedlichen Positionen angeordnet sind und die voneinander unterschiedliche Abtastrichtungen des Laserlichts aufweisen, und wobei die zweite geneigte Linse zur optischen Achse des optischen Bestrahlungssystems oder des zweiten Objektivlinsensystems zum Beseitigen einer am zweiten Drehpunkt verbleibenden astigmatischen Abweichung geneigt angeordnet ist.

13. Laser-Abtastophthalmoskop nach Anspruch 11 oder 12, wobei die Linsenbefestigung zumindest zwei geneigte Linsen aufweist, die in zueinander umgekehrten Phasen in Bezug auf die optische Achse zum Aufheben einer Neigung einer Bildebene auf einer Augenhintergrund-Konjugatebene angeordnet sind.

14. Laser-Abtastophthalmoskop, umfassend:

ein optisches Bestrahlungssystem, das eine Abtasteinheit zum Abtasten eines Augenhintergrunds eines Auges einer Person mit von einer Lichtquelle emittiertem Laserlicht und ein Objektivlinsensystem umfasst, das zwischen dem Auge des der Person und der Abtasteinheit zum Bilden eines Drehpunkts angeordnet ist, um den herum sich das von der Abtasteinheit geführte Laserlicht als Reaktion auf eine Betätigung der Abtasteinheit dreht; und
ein optisches Lichtaufnahmesystem, welches das Objektivlinsensystem mit dem optischen Bestrahlungssystem gemeinsam verwendet, und das ein Lichtaufnahmeelement zum Aufnehmen von Licht vom mit dem Laserlicht bestrahlten Augenhintergrund aufweist,
wobei die Abtasteinheit zwei optische Scanner aufweist, die an voneinander verschiedenen Positionen angeordnet sind und die voneinander verschiedene Abtastrichtungen des Laserlichts aufweisen,
wobei das Objektivlinsensystem ein erstes Linsensystem zum Beugen des Laserlichts zum Drehpunkt hin und ein zweites Linsensystem umfasst, das zwischen dem ersten Linsensystem und der Abtasteinheit angeordnet ist,
**dadurch gekennzeichnet, dass** das zweite Linsensystem eine geneigte Linse aufweist, die zu einer optischen Achse des optischen Bestrahlungssystems geneigt angeordnet ist, und die geneigte Linse zur optischen Achse des optischen Bestrahlungssystems zum Aufheben einer durch die zwei optischen Scanner verursachten astigmatischen Abweichung am Drehpunkt geneigt angeordnet ist.

**Revendications**

1. Ophtalmoscope laser à balayage (1) comprenant :

un système optique d'irradiation (10) qui comporte une unité de balayage (16) pour balayer un fond de l'oeil d'un sujet avec une lumière laser émise par une source de lumière (11), et un système de lentille d'objectif (17) qui est disposé entre l'oeil du sujet et l'unité de balayage de manière à former un point de pivotement autour duquel la lumière laser guidée à partir de l'unité de balayage pivote en réponse à un fonctionnement de l'unité de balayage ; et

un système optique de réception de lumière (20) qui partage le système de lentille d'objectif avec le système optique d'irradiation, et qui a un élément de réception de lumière (25, 27, 29) pour recevoir de la lumière provenant du fond d'oeil irradié avec la lumière laser,

dans lequel le système de lentille d'objectif comporte un premier système de lentille (17a) pour courber la lumière laser vers le point de pivotement et un deuxième système de lentille (17b) disposé entre le premier système de lentille et l'unité de balayage, et

**caractérisé en ce que** le deuxième système de lentille a une lentille inclinée (172, 173) disposée de manière à être inclinée vers un axe optique du système optique d'irradiation, et une surface de lentille (172a, 173a) sur le côté de la source de lumière dans la lentille inclinée a un rayon de courbure supérieur à celui d'une surface de lentille sur le côté de la source de lumière dans le premier système de lentille.

2. Ophtalmoscope laser à balayage selon la revendication 1, dans lequel le deuxième système de lentille corrige une aberration provoquée par le premier système de lentille.

3. Ophtalmoscope laser à balayage selon la revendication 2, dans lequel
la source de lumière émet au moins la lumière laser ayant une première longueur d'onde et une lumière laser ayant une deuxième longueur d'onde différente de la première longueur d'onde, et
le deuxième système de lentille comporte une lentille collée qui corrige une aberration chromatique provoquée par le premier système de lentille en ce qui concerne au moins la lumière laser ayant la première longueur d'onde et la lumière laser ayant la deuxième longueur d'onde.

4. Ophtalmoscope laser à balayage selon la revendication 3, dans lequel les indices de réfraction d'une pluralité de lentilles incluses en outre dans la lentille collée et un adhésif pour relier la pluralité de lentilles les unes aux autres ont la même valeur.

5. Ophtalmoscope laser à balayage selon l'une quelconque des revendications 1 à 4, dans lequel le premier système de lentille est disposé de sorte qu'un axe optique du premier système de lentille s'étende le long d'un axe optique du système optique d'irradiation.

6. Ophtalmoscope laser à balayage selon l'une quelconque des revendications 1 à 5, dans lequel le premier système de lentille comporte au moins une lentille qui est disposée de manière à être inclinée vers l'axe optique du système optique d'irradiation dans une direction pour annuler une inclinaison d'un plan d'image sur un plan conjugué de fond d'oeil qui est provoquée par la lentille inclinée.

7. Ophtalmoscope laser à balayage selon l'une quelconque des revendications 1 à 6,
dans lequel le premier système de lentille est une lentille asphérique qui limite une aberration sphérique de formation d'image au niveau du point de pivotement en formant au moins une surface de lentille de celle-ci dans une forme asphérique.

8. Ophtalmoscope laser à balayage selon l'une quelconque des revendications 1 à 7,
dans lequel le deuxième système de lentille est disposé entre un plan d'image intermédiaire formé le plus proche du premier système de lentille dans un plan d'image intermédiaire du fond d'oeil qui est formé en utilisant le système de lentille d'objectif, et le premier système de lentille.

9. Ophtalmoscope laser à balayage selon l'une quelconque des revendications 1 à 8, dans lequel
l'unité de balayage a deux dispositifs de balayage optique qui sont disposés à des positions mutuellement différentes et qui ont des directions de balayage mutuellement différentes de la lumière laser, et
la lentille inclinée est disposée de manière à être inclinée vers l'axe optique du système optique d'irradiation dans une direction pour annuler une différence astigmatique au niveau du point de pivotement qui est provoquée par les deux dispositifs de balayage optique.

10. Ophtalmoscope laser à balayage selon l'une quelconque des revendications 1 à 9, comprenant en outre :

un boîtier qui loge au moins une partie du système optique d'irradiation et au moins une partie du système optique de réception de lumière ; et
une fixation de lentille montée sur une surface du boîtier au niveau d'un côté du sujet,
dans lequel le système de lentille d'objectif est disposé dans la fixation de lentille.

**11.** Ophtalmoscope laser à balayage selon l'une quelconque des revendications 1 à 9, comprenant en outre :

un boîtier qui loge au moins une partie du système optique d'irradiation et au moins une partie du système optique de réception de lumière ; et

une fixation de lentille qui commute un angle de champ d'imagerie en fonction de l'attachement à une surface du boîtier au niveau d'un côté du sujet ou du détachement de celle-ci, et qui a un deuxième système de lentille d'objectif pour courber la lumière laser incidente par l'intermédiaire du système de lentille d'objectif vers un deuxième point de pivotement différent du point de pivotement,

dans lequel le deuxième système de lentille d'objectif comporte au moins une deuxième lentille inclinée disposée de manière à être inclinée vers l'axe optique.

**12.** Ophtalmoscope laser à balayage selon la revendication 11,

dans lequel l'unité de balayage a deux dispositifs de balayage optique qui sont disposés à des positions mutuellement différentes et qui ont des directions de balayage mutuellement différentes de la lumière laser, et

dans lequel la deuxième lentille inclinée est disposée de manière à être inclinée vers l'axe optique du système optique d'irradiation ou du deuxième système de lentille d'objectif pour annuler une différence astigmatique restant au niveau du deuxième point de pivotement.

**13.** Ophtalmoscope laser à balayage selon la revendication 11 ou 12,

dans lequel la fixation de lentille a au moins deux lentilles inclinées disposées dans des phases mutuellement inverses par rapport à l'axe optique de manière à annuler une inclinaison d'un plan d'image sur un plan conjugué de fond d'oeil.

**14.** Ophtalmoscope laser à balayage comprenant :

un système optique d'irradiation comportant une unité de balayage pour balayer un fond de l'oeil d'un sujet avec une lumière laser émise par une source de lumière, et un système de lentille d'objectif qui est disposé entre l'oeil du sujet et l'unité de balayage de manière à former un point de pivotement autour duquel la lumière laser guidée à partir de l'unité de balayage pivote en réponse à un fonctionnement de l'unité de balayage ; et

un système optique de réception de lumière qui partage le système de lentille d'objectif avec le système optique d'irradiation, et qui a un élément de réception de lumière pour recevoir de la lumière provenant du fond d'oeil irradié avec la lumière laser,

dans lequel l'unité de balayage a deux dispositifs de balayage optique qui sont disposés à des positions mutuellement différentes et qui ont des directions de balayage mutuellement différentes de la lumière laser,

dans lequel le système de lentille d'objectif comporte un premier système de lentille pour courber la lumière laser vers le point de pivotement et un deuxième système de lentille disposé entre le premier système de lentille et l'unité de balayage, et

**caractérisé en ce que**

le deuxième système de lentille a une lentille inclinée disposée de manière à être inclinée vers un axe optique du système optique d'irradiation, et la lentille inclinée est disposée de manière à être inclinée vers l'axe optique du système optique d'irradiation pour annuler une différence astigmatique au niveau du point de pivotement qui est provoquée par les deux dispositifs de balayage optique.

*Fig. 1*

Fig. 2

**Fig. 3**

**Fig. 4**

**Fig. 5**

Fig. 6

*Fig. 7*

*Fig. 8*

EP 3 235 421 B1

*Fig. 9*

*Fig.10*

```
                    ┌─────────────┐
                    │    START    │
                    └──────┬──────┘
                           │
S1   ┌─────────────────────▼─────────────────────┐
     │            SELECT IMAGING MODE             │
     └─────────────────────┬─────────────────────┘
                           │
S2                    ◇────▼────◇              No
              ╱ IS SPECIFIC MODE ╲──────────────────────┐
              ╲    SELECTED?     ╱                       │
                    ◇────┬────◇                          │
                      Yes │                              │
S3   ┌─────────────────────▼──────────────┐   S8  ┌──────▼─────────────────────┐
     │ RESTRICT ADJUSTMENT RANGE OF       │       │    ADJUST LIGHT INTENSITY  │
     │ LIGHT INTENSITY AND/OR GAIN        │       │       AND/OR GAIN          │
     └─────────────────────┬──────────────┘       └──────┬─────────────────────┘
S4   ┌─────────────────────▼──────────────┐   S9  ┌──────▼─────────────────────┐
     │     ADJUST LIGHT INTENSITY         │       │    CAPTURE FUNDUS IMAGE     │
     │        AND/OR GAIN                 │       └──────┬─────────────────────┘
     └─────────────────────┬──────────────┘              │
S5   ┌─────────────────────▼──────────────┐              │
     │       CAPTURE FUNDUS IMAGE         │              │
     └─────────────────────┬──────────────┘              │
S6   ┌─────────────────────▼──────────────┐              │
     │      CAPTURE ARTIFACT IMAGE        │              │
     └─────────────────────┬──────────────┘              │
S7   ┌─────────────────────▼──────────────┐              │
     │   ACQUIRE DIFFERENCE IMAGE         │              │
     │  BETWEEN FUNDUS IMAGE AND          │              │
     │        ARTIFACT IMAGE             │               │
     └─────────────────────┬──────────────┘              │
                           │◄───────────────────────────┘
                    ┌──────▼──────┐
                    │     END     │
                    └─────────────┘
```

*Fig. 11*

*Fig. 12*

*Fig. 13*

*Fig. 14*

Fig. 15

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2901919 A
- JP 2016028687 A